# EUROPEAN PATENT SPECIFICATION

(11) **EP 3 068 410 B1**
(45) Date of publication and mention of the grant of the patent: **24.10.2018**
(21) Application number: 14798838.0
(22) Date of filing: 13.11.2014
(51) Int. Cl.: A61P 17/02, A61P 9/00, A61P 25/00, A61P 37/00, A61P 29/00

(54) **METHOD FOR OBTAINING A CYTOKINE-RICH COMPOSITION AND COMPOSITION OBTAINED BY MEANS OF THIS METHOD**
VERFAHREN ZUR HERSTELLUNG EINER ZYTOKINREICHEN ZUSAMMENSETZUNG UND MIT DIESEM VERFAHREN ERHALTENE ZUSAMMENSETZUNG
PROCÉDÉ POUR OBTENIR UNE COMPOSITION RICHE EN CYTOKINE ET COMPOSITION OBTENUE AU MOYEN DE CE PROCÉDÉ

(30) Priority: 14.11.2013 EP 13192928
(43) Date of publication of application: 21.09.2016
(73) Proprietor: NTE-SENER HEALTHCARE, S.A., 08290 Cerdanyola del Valles (ES)
(72) Inventor: VIDAL FAYOS, Francisco, E-08339 Vilassar de Dalt (ES)
(74) Representative: ZBM Patents - Zea, Barlocci & Markvardsen
(86) International application number: PCT/EP2014/074493
(87) International publication number: WO 2015/071364

(56) References cited:
- EP-A1- 2 133 086
- WO-A1-00/62828
- WO-A1-2010/118979
- WO-A2-2007/027178
- WO-A2-2009/052221
- ISOGAI NORITAKA ET AL: "Cytokine-rich autologous serum system for cartilaginous tissue engineering.", ANNALS OF PLASTIC SURGERY JUN 2008, vol. 60, no. 6, June 2008 (2008-06), pages 703-709, XP009176386, ISSN: 1536-3708 cited in the application

## Description

The present invention relates to a method or methods for obtaining a serum rich in cytokines and/or growth factors (including other biological mediators), as well as those serum, which have utility for treating pathological conditions caused by trauma, ischemia, inflammation and degeneration in general and for tissue repair and regeneration, in particular. Therefore, the invention could be framed in the field of biomedicine.

### BACKGROUND ART

Many adult animals are able to regenerate complex body structures after tissue injury, and among vertebrates it is known that amphibians have a greater capacity to respond to these regenerative processes compared with mammals in which regeneration appears to be significantly reduced.

The repair and regeneration of all or part of organs and tissues is a common process that occurs in response to multiple pathologies such as trauma, ischemia, acute and chronic inflammation and degenerative processes. Repair and regeneration of damaged tissue is a complex biological process initially dependent on activation, signaling and mobilization of cells by means of interrelated, sequential and/or parallel, multiple molecules, mediators and other signaling or induction factors. Although progress has been made on the study of molecular mechanisms and signaling pathways that regulate regeneration processes, it is not yet exactly known why some tissues and organs regenerate properly while others do not.

While knowledge of the signaling pathways that control different regenerative processes is still incomplete, it is clear that there is a significant overlap in the pathways involved. However, it is likely that the precise function of signals in different regeneration systems be as diverse as the cellular events that occur. Future research will undoubtedly increase not only the list of new elements involved, but also reveal differences in their role in other different systems.

Depending on factors inherent to pathological processes themselves, the repair and regeneration activity may not be optimal. In fact in most cases it is not. Examples are scarring or fibrosis areas known as processes that occur after trauma, ischemia, inflammation and degeneration. Within this context therapies have directed their efforts not only to prevent and treat the causes but also to promote and facilitate healing (repair and regeneration of damaged tissue and organ) trying to minimize the negative effects of a repair or regeneration sub-optimum. Among other biologics, current therapies include various hormones, steroids, HG, EPO, G-CSF, GM-CSF, M-CSF, IL-2, IL-3, and BMP, currently used in organ and tissue transplants, and in regenerative medicine to recover injured or damaged tissues.

Peripheral blood is fully involved in any hemostatic, inflammatory, reparative and regenerative process. Peripheral blood cells (including erythrocytes, but mainly platelets and leukocytes) have a central role in regenerative reparative activity. Platelets exert a primary role in hemostasis injury. Platelets behave also a high activity in initiating reparative and regenerative cycles, by synthesizing and releasing molecules once fibrinolysis occurs. These molecules, in turn, activate other cells such as leukocytes creating successive biological functions ("regenerative cycle") designed to repair and regenerate damaged tissue.

With the aim of promoting regeneration of tissues, as well as for the control of hemostasis and inflammatory processes, blood products generically defined as blood or blood derivatives have been employed. Examples of these products include blood plasma, blood serum, leukocyte concentrate, mononuclear cells, or platelet-rich fibrin gels (PRF). Among these, the plasma known as Platelet Rich Plasma (PRP) has been widely used when it became apparent some improvement in postoperative conditions and in inflammation and pain associated with certain acute and chronic processes. Nonetheless, poor therapeutic effects have been demonstrated in a meaningful way.

Many reasons can influence in the low therapeutic response of PRP, such as their preparation, the dose employed, the formulation, the application site, the time of administration, the cellular components contained in the PRP, the activation degree of biological responses associated to the clotting mechanisms and to the inflammatory process, as well as the health condition and genetic fingerprint of the donor and recipient.

Examples of PRP and methods of preparation are disclosed in the document US 20050252867. This document discloses different methods of obtaining PRP from blood containing anticoagulants. In US20050252867 the anti-coagulated blood is centrifuged to obtain the PRP. Part of this PRP is then let to clot to obtain a serum comprising thrombin, which is used as an additive of the previously obtained PRP.

In several cases, these blood derivatives are prepared in situ from the blood of the subject being treated, for example, in an operating room. This implies that the extracted blood has to be managed as quickly as possible. Nonetheless, many of the processes for obtaining the blood derivatives imply long processing times, which means that in case of being needed in a programmed surgical intervention, blood of the subject has to be extracted and treated to obtain the derivative with enough time. Obviously, this cannot be possible if the subject is being treated in emergency rooms.

On the other hand, a quick obtention of the blood derivative (PRP, PRF, etc.) gives rise to products that may not contain all the molecules or the effective amounts of them. Thus, the treatment with these blood preparations is sometimes not as effective as desired or expected.

Other blood derivatives being studied with the purpose of inducing also tissue regeneration or promoting healing of diseases are the blood serums. An example of these may be the cytokine-rich serum disclosed by Isogai et al. in "Cytokine-Rich Autologous Serum System for Cartilaginous Tissue Engineering", Annals of Plastic Surgery - 2008, Vol. NO. 60, pp.: 703-709".

Isogai et al. disclose a method for obtaining a serum useful in the regenerative process of cartilaginous tissue, and they exemplify how a canine auricular cartilage can be reconstructed or regenerated. The cytokine-rich serum is obtained by extracting blood from the dogs, mixing the whole blood in a bag containing glass beads, and shaking the mixture at 30 rpm/min for 1 hour. The platelets of the blood adhere to the adsorbed plasma proteins like fibrinogen and fibronectin disposed on the glass beads and the applied rotation causes shear-induced stimulation and activation of the platelets. The activated blood is then centrifuged at 3000 rpm to obtain a precipitate containing fibrin with beads, and a supernatant which is a cytokine-rich autologous serum.

One disadvantage associated with the method of Isogai et al. is that the presence for instance of the glass beads increases the risk of activating the immune cells present in the treated blood. This activation may lead to deregulation of the immune system of the recipient once the blood derivative is finally administered, since this derivative may contain in several proportions, those components informing the immune system of the recipient that it has to be activated too. An aberrant or nor proper activation of the immune system implies the risk of allergic reactions or even the development of autoimmune diseases. In addition, preparation of the cytokine-rich serum of Isogai et al. involves the performance of technically complex steps that lengths the process for obtaining the serum and makes it not applicable in situ, for example, in the operating room.

Finally, other blood derivatives proposed as sealants or as regenerative tissue agents are the so-called platelet-rich fibrins (PRF). The PRF is formed when whole blood collected without anticoagulant is let to coagulate at the same time it is centrifuged. Three phases are separated: (1) a coagulated red cell layer; (2) a rigid and elastic PRF gel, that once squeezed allows obtaining a fibrin membrane for clinical applications; and (3) a liquid supernatant serum, generally discarded. Some documents disclose that the releasate (liquid) of this PRF fraction contain growth factors increasing slightly during a period comprised from 1 to 2 hours after extraction of the whole blood. An example of this is proposed by Su et al. in "In vitro release of growth factors from platelet-rich fibrin (PRF): a proposal to optimize the clinical applications of PRF", Oral Surg Oral Med Oral Pathol Oral radiol Endod - 2009, Vol. No. 108, pp.: 56-61. Su et al. suggest using PRF within the first hour after its obtention and without discarding the releasate, since they observed that growth factors could be released during this period of time from the fresh PRF. The document proposes then using the PRF as well as the releasate in therapeutical applications.

Another critical point in the regeneration of tissues by means of blood derivatives, especially when fibrin clots (PRF) or gels are used is the time and method of administration of these products. In surgery, for example, fibrin clots or gels are applied in the damaged area when the process of forming the primary hemostatic or local platelet plug has already started. This gives a sealant effect due to adhesion to the tissues (vascular and connective) of the affected area. However, the signaling to other cells is restricted, having a local paracrine effect and sometimes therapeutic ineffectiveness.

Therefore, although several blood derivatives are available to be administered in subjects in need of tissue repair or regeneration, or in need of sealant compositions in operating rooms, it is necessary to design one or more products, easy and fast to be obtained, and that contain high amounts of effective cytokines and other growth factors, found naturally in reparative and regenerative processes.

### SUMMARY OF THE INVENTION

The inventors determined that using isolated whole blood or other biological samples containing platelets and/or leukocytes, and inducing clot formation, the clot could then be stressed by means of a method that gives raise to serums highly enriched with cytokine and other growth factors, as well as comprising coagulation factors. The method for obtaining the serums includes a first mild activation of the platelets and/or leukocytes contained in the clot, and further steps, in which strong activation of platelets and/or leukocytes is induced, said strong activation being in particular performed by means of at least two additional activation steps, or one additional step with an increasing along time gravity force.

The invention is defined in the claims.

Thus, in a first aspect the invention relates to a method for preparing a cytokine-rich serum, or which is the same, a serum comprising cytokines and coagulation factors, the method comprising the following steps:
(a) Submitting an isolated biological sample comprising platelets and/or leukocytes to a gravity force comprised from 100 g to 200 g to obtain a clot and supernatant;
(b) Submitting only the clot or, alternatively, the clot and supernatant of step (a) to a gravity force comprised from 400 g to 2500 g to obtain a supernatant SN1; wherein the gravity force is discontinuously applied and carried out by means of:
   (i) centrifuging the clot of step (a), or alternatively the clot and supernatant, at a gravity force comprised from 400 g to 500 g;
   (ii) recovering the supernatant of step (i), which constitutes partially the supernatant SN1ii;
   (iii) centrifuging the remaining clot at a gravity force comprised from 1500 g to 2500 g and recovering the supernatant of step (iii), which constitutes partially the supernatant SN1iii of step (b), constituting the supernatants of steps (ii) and (iii) the entire supernatant SN1; and
(c) Recovering said supernatant SN1 of step (b), which is a serum comprising cytokines and coagulation factors,
wherein the gravity force is applied by a process selected from the group consisting of centrifugation and physical-suction.

With this method, activation of the platelets and/or leukocytes is achieved due to the fact that the gravity force puts each cell in contact with each other, thus allowing starting the signalling processes between them. In addition, a high gravity force promotes the breaking of the cells and the release of the contents in its granules. The gravity force is the leading cause of the process, and it can be applied to the biological sample by several means and, in particular by centrifugation. Indeed, each of the steps (a) and (b) can be carried out in order to separate the cells in the biological sample, to activate the coagulation cascade and to break the fibrin lattice (causing fibrinolysis). The different gravity forces applied in each step, allow obtaining different cytokines and growth factors along time.

As will be illustrated in the Examples below, the gravity force pattern applied, with the several embodiments or versions encompassed herein, allows a final strong activation of platelets and/or leukocytes that has the advantage of providing serums with high levels of cytokines and growth factors. Advantageously, these high levels of cytokines and growth factors in the serums are obtained in absence of any artificial coagulation contact phase activator of platelets (such as glass, glass-beads, kaolin, and ellagic acid)

According to the inventors knowledge, this is the first time that is being defined a system in which fibrinolysis is performed by gravity force (by physical means). Other approaches of prior art include the use of chemical or biochemical means.

Along this description the gravity force is in particular applied by means of centrifugations. However, the invention also encompasses other equivalent physical or chemical means (such physical-suction or collagen contact) that lead to the same effect, namely to the activation of cells.

As above announced, the steps and sequence of steps of the proposed method of the invention unexpectedly conduct to a serum with high concentrations of cytokines. This serum is able to promote tissue repair in a greater degree than other blood derivatives. In addition, the method for obtaining the serums can be performed quickly, if needed, without compromising the quality and effect of the serums. Thus, the method for producing these serums can be performed in operating rooms or meanwhile the subject is being treated with other means. By this way, the method allows the obtention of an autologous cytokine-rich serum (or serum comprising cytokines and coagulation factors). The serum is highly effective in terms of treatment, because it contains higher concentrations of cytokines and of other molecules than the produced in naturally phenomena when for example, the coagulation cascade and other processes are activated.

Thus, as shown in the examples below, the method of the invention provides serums and other blood derivatives with higher concentrations of cytokines, and of other biological mediators than present in the starting sample. Also a higher cellular response is achieved with these serums. Data shown in Tables 1 and 2, and FIG.s show that concentration of certain cytokines increase and even there appear new cytokines, growth factors and other biological mediators of the cellular response. Among the cytokines (including growth factors) that experienced a significant increase when the method of the invention is applied, there are found for example, transforming growth factor beta (TGF-β 1), Hepatocyte Growth Factor (HGF), epidermal growth factor (EGF), Vascular Endothelial Growth Factor (VEGF), interleukin-6 (IL-6), interleukin -8 (IL-8), Interleukin 10 (IL-10) Derived Growth Factor P (PDGF), Factor VII, von Willebrand Factor. All of them reach levels with a valuable therapeutically potential. The presence of all these molecules is the result of the activation and signaling of the cells in the clot, said activation promoted by the method of the invention.

The preparation of serums of the present invention in *"ex vivo"* conditions is carried out in an environment not comprising the conditioning factors of an *"in vivo"* behavior. This promotes the activation of the synthesis and of the release of multiple proteins and other molecules, which achieve high concentrations and that in turn enable further *"ex vivo"* cells activation. Moreover, this high concentration of the desired molecules enables that when the serum is administered to a subject, those *"in vivo"* cell populations be activated at the site of administration. Even by means of an enhanced signaling biological, activation of far-related cells may be activated and recruited.

Moreover, thanks to the speed in which the method can be performed, the serum (which can be autologous or allogeneic) includes those cytokines and coagulation factors of short shelf life, but of great interest for the therapeutically aim.

Therefore, the final objective of the present invention is to provide a new method to achieve different types of serum and other derivatives whose characteristics and initial contents, trigger and/or modulate a biological response and thereby exert a therapeutic role in the cycle of tissue repair and regeneration.

In summary the method of the invention allows producing regenerative and inductive biological factors (cytokines, such as interleukins, growth factors, etc.) from isolated samples of a subject through *"ex vivo"* physical and/or biochemical methods. All these molecules can be topically or systemically re-administered in an allogeneic or autologous manner. It is especially advantageous to use the serum obtained by the method in an autologous manner, thereby minimizing possible side-effects, such as rejection or inflammatory reactions.

Another aspect of the invention is therefore a serum comprising cytokines and coagulation factors obtainable by the method disclosed above.

This serum comprises significant concentrations of cytokines (including growth factors) and coagulation factors selected from the group consisting of:
- Transforming growth factor beta (TGF-β 1), which concentration in the final serum is comprised from 30.0 ng/ml to 50.0 ng/ml;
- Hepatocyte Growth Factor (HGF), which concentration in the final serum is comprised from 3.0 ng/ml to 6.0 ng/ml;
- Epidermal growth factor (EGF), which concentration in the final serum is comprised from 65.0 pg/ml to 80.0 pg/ml;
- Vascular Endothelial Growth Factor (VEGF), which concentration in the final serum is comprised from 65.0 pg/ml to 80.0 pg/ml
- Interleukin 10 (IL-10), which concentration in the final serum is comprised from 5.0 pg/ml to 10.0 pg/ml;
- Derived Growth Factor P (PDGF), which concentration in the final serum is comprised from 35.0 ng/ml to 45.0 ng/ml;
- Factor VII, which concentration in the final serum is comprised from 170.0 U/dl to 250.0 U/dl, wherein U means enzymatic units;
- von Willebrand Factor, which concentration in the final serum is comprised from 100.0 U/dl to 150.0 U/dl; and mixtures thereof.

The serum is capable of inducing proliferation of bone marrow cells in such a way that, starting from a culture of bone marrow cells seeded in a Petri dish with a density comprised from 150 cells /cm² to 250 cells /cm², by addition of the serum in a percentage by weight comprised from 10 % to 30 % in the final culture, the amount of hematopoietic cells identified by the membrane marker CD34 is comprised from 1.0 x 10⁶ to 1.5 x 10⁶ measured 15 days after contacting the bone marrow cells with the serum. The culture medium supplemented with an amount of serum comprised from 10 % to 30 % w/w may be any of the known by the skilled man in which bone marrow cells can grow. An example is a Dulbecco's modified medium comprising glucose and having 4 mM L-Glutamine, and 0.05 units of penicillin and 0. 05 g of streptomycin.

The advantages of this serum have been mentioned previously while defining the method of the invention. It is to be emphasized that the serums are also conceivable as autologous serums obtainable in *"ex vivo"* conditions, in other words, from an isolated sample of a subject, which is then processed to finally obtain a composition with cytokines and coagulation factors that can be administered to the same subject.

It is also part of the invention a process for preparing a fibrin gel composition comprising platelets and/or leukocytes embedded in a fibrin matrix, said gel comprising also a serum with cytokines and coagulation factors in liquid form disposed in-between the fibrin matrix, said fibrin gel obtainable by the process disclosed above for the preparation of a serum comprising cytokines and coagulation factors, and further comprising mixing the supernatant SN1 of step (c) with a composition comprising fibrinogen, platelets and/or leukocytes selected from the group consisting of a platelet-rich plasma, a platelet concentrate, a leukocyte-rich plasma, a leukocyte concentrate, a platelet-poor plasma, a plasma concentrate and mixtures or combinations thereof; and adding to the mixture of previous step an ionic calcium source to adjust the ionic calcium to a final concentration comprised from 1.0 µmol/ml to 50.0 µmol/ml to obtain a second clot, which is a fibrin gel composition comprising platelets and/or leukocytes embedded in a fibrin matrix, said gel comprising also a serum with cytokines and coagulation factors in liquid form disposed in-between the fibrin matrix.

It is also part of the invention a fibrin gel composition comprising platelets and/or leukocytes embedded in a fibrin matrix, said gel comprising also a serum with cytokines and coagulation factors in liquid form disposed in-between the fibrin matrix obtainable by the process disclosed above.

It is also another part of the invention a process for preparing another serum comprising cytokines and coagulation factors, also named throughout this description supernatant SN2, said serum obtainable by the process disclosed above for the preparation of a fibrin gel composition; and further submitting this fibrin gel to physical and/or chemical and/or biochemical means to break the fibrin matrix by these means to obtain a supernatant SN2; and recovering the supernatant SN2, which is a serum comprising cytokines and coagulation factors.

Thus, it is also part of the invention a supernatant SN2, which is a serum comprising cytokines and coagulation factors obtainable by the process disclosed above.

Another aspect of the invention is a pharmaceutical composition, comprising any of the serums as defined above, or the fibrin gel composition as defined above, or mixtures thereof, together with appropriate amounts of pharmaceutical acceptable excipients and/or carriers.

It is also an aspect of the invention any of the serums comprising cytokines and coagulation factors as defined above, or alternatively a fibrin gel composition as defined above or, alternatively, a pharmaceutical composition as defined above for use as a medicament.

Thus, although the present invention discloses methods of treatment in which the serums of the invention, or alternatively the fibrin gel composition or the pharmaceutical compositions as defined above are applied to a subject in need thereof, they are not claimed.

The invention also encompasses as an aspect an *ex vivo* organ perfusion liquid composition comprising as an ingredient any of the serum as defined above or, alternatively, the pharmaceutical compositions as defined above, or mixtures thereof.

Another aspect of the invention is any of the serums as defined above, or a fibrin gel composition as defined above, or a pharmaceutical composition as defined above, or mixtures thereof, for use as an *in vitro,* and/or *ex vivo* and/or *in vivo* cell proliferative and/or cell differentiating agent. This means that the serums, the fibrin gel composition or the pharmaceutical compositions comprising them are for use as an agent supporting the tissue regeneration process by the aid of the cytokines and coagulation factors contained in them.

In another aspect, the invention also relates to the serums as defined above, or a fibrin gel composition as defined above, or a pharmaceutical composition as defined above, or mixtures thereof, for use as agents inducing *in vitro,* and/or *ex vivo* and/or *in vivo* cytokine cell synthesis. This means that cytokine cell synthesis is promoted, initiated or enhanced.

All these effects of the serums, or of the fibrin gel composition, performed on cells of different types (other platelets, leukocytes, fibroblasts...) have the aim of obtaining compositions with a higher treating capability. For example, if the synthesis of cytokine is induced, the *ex vivo* composition or the *in vivo* environment are both equivalent to a situation in which, the natural in the body repairing system was highly incentivized, stimulated or activated. This allows a better and faster healing process than the natural occurring. This is in special advantageous for subjects whose system involving the components comprised in the serums or compositions of the invention (cytokines and coagulation factors) is compromised (i.e. immunodeficient subjects, subjects with haemophilia, etc.).

### BRIEF DESCRIPTION OF THE DRAWINGS

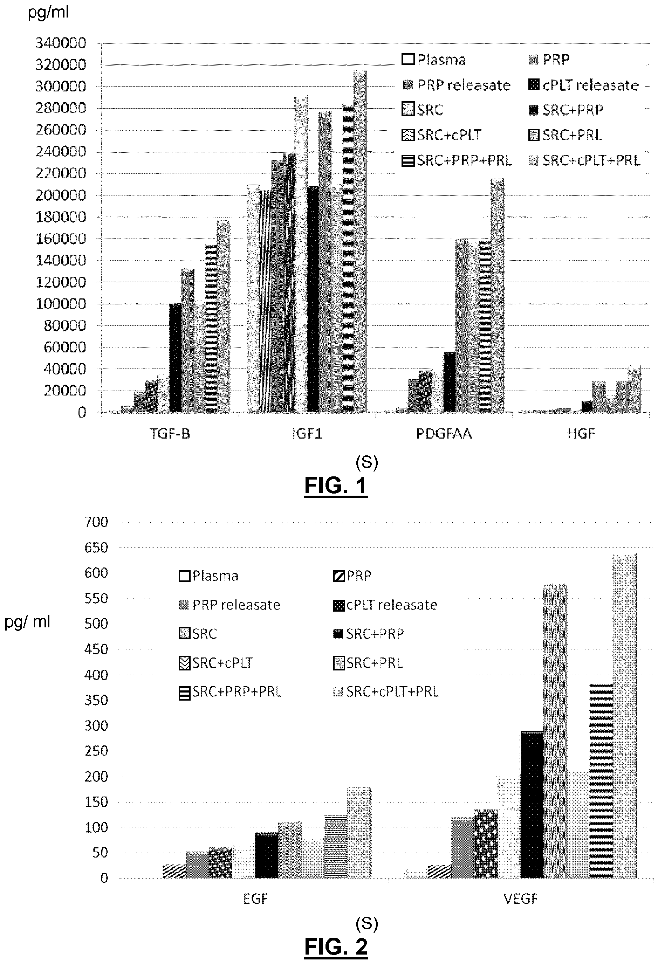

FIG. 1 is a bar-diagram that shows in the Y-axis the amounts (pg/ml) of TGF-β, IGF-1 and HGF and PDGFAA in different samples (S) listed in the X-axis. The term "Releasate" is described as the liquid or the resulting from activation of platelets obtained with calcium chloride and the formation of clot degradation. PRP: Platelet Rich Plasma; SRC: Cytokine-rich Serum; PRL: Leukocyte-rich plasma; cPLT: Platelet concentrate.
FIG. 2 is another bar-diagram showing in the Y-axis the amounts of EGF and VEGF in the different samples (S) listed in the X-axis. The term "Releasate" is described as the liquid or the supernatant resulting from activation of platelets obtained with calcium chloride and the formation of clot degradation. PRP: Platelet Rich Plasma; SRC: Cytokine-rich Serum; PRL: Leukocyte-rich plasma; cPLT: Platelets concentrate.

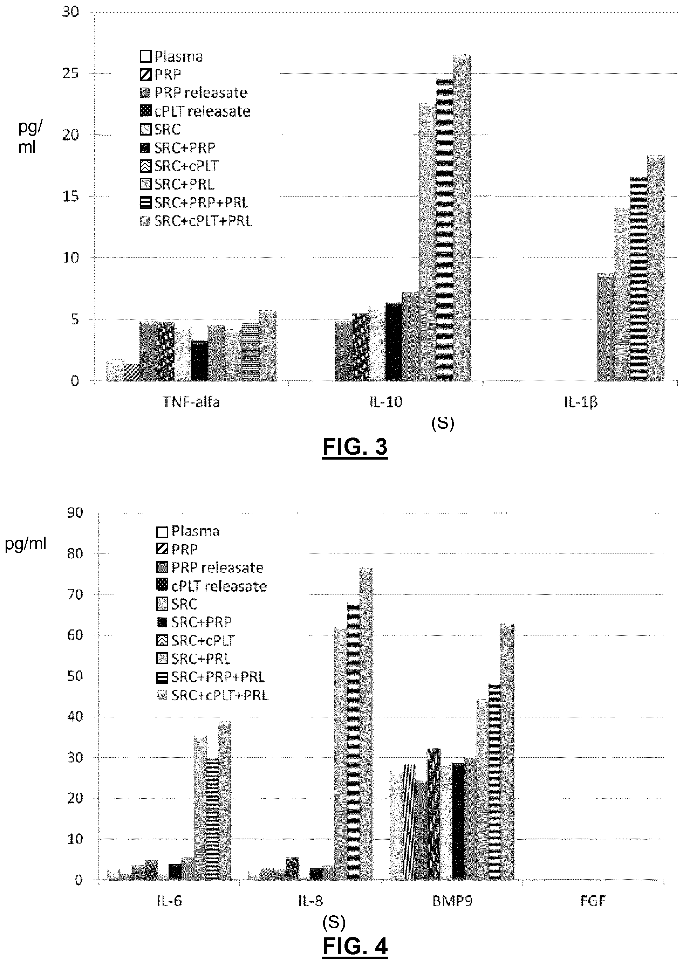

FIG. 3 shows the amounts (pg/ml) in the Y-axis of TNF-alpha, IL-10 and IL-1β in the different samples (S) listed in the X-axis. Abbreviations and the term "Releasate" have the same meaning as indicated for FIGs 1 and 2.
FIG. 4 shows the amounts (pg/ml) in the Y-axis of IL-6, IL-8, BMP-9 and FGF in the different samples (S) listed in the X-axis. Abbreviations and the term "Releasate" have the same meaning as indicated for FIGs 1 and 2.

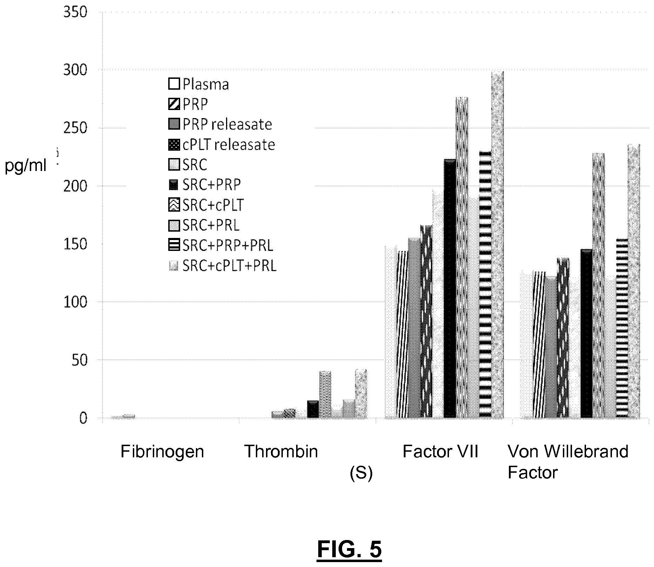

FIG. 5 shows the amounts (pg/ml) in the Y-axis of fibrinogen, thrombin, Factor VII and Von Willebrand Factor in the different samples (S) listed in the X-axis. Abbreviations and the term "Releasate" have the same meaning as indicated for FIGs 1 and 2.

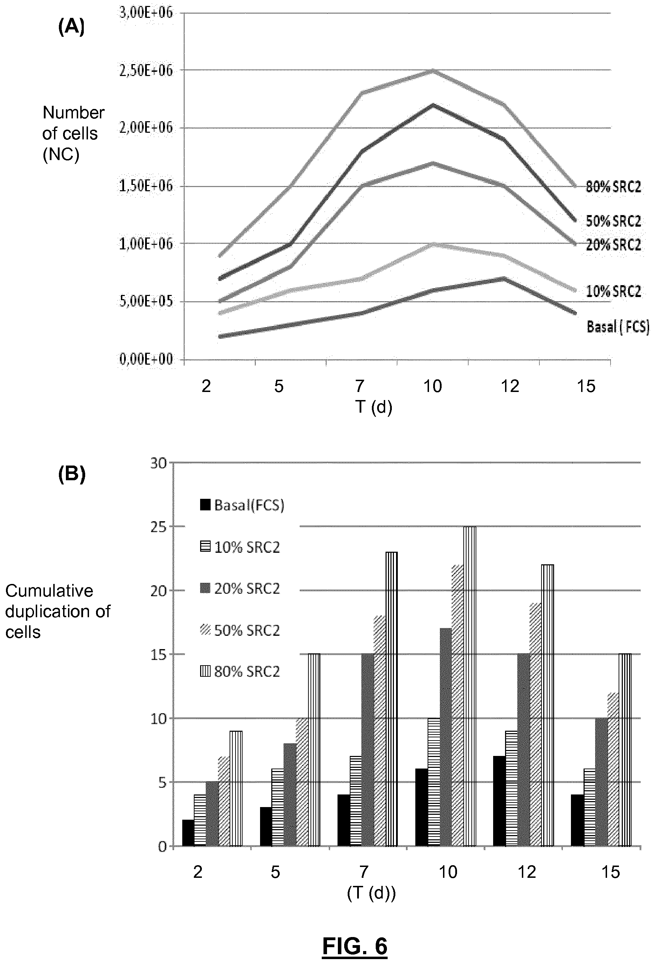

FIG. 6 is a graphic that represents the number of bone marrow cells (Y-axis, number of cells; NC) at various times (T in days, d) growing with a basal media supplemented with fetal calf serum (FCS or Foetal calf serum) or supplemented with different concentrations ((10, 20, 50 and 80% w/w) of a cytokine-rich serum, SRC2, of the invention. Panel (A) shows the number of cells along time in the different tested conditions (different % of the serum of the invention). In Panel (B) it is shown the cumulative duplication of cells (CDC) contacted with the different mediums.

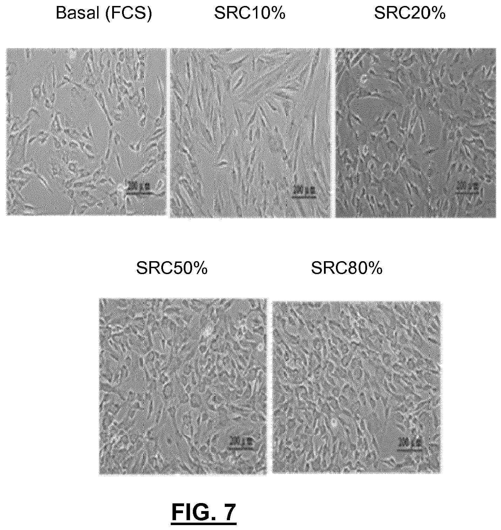

FIG. 7 shows different optical microscopic images (100X) of the bone marrow cells cultivated as indicated in FIG. 6; cells in basal medium supplemented with fetal calf serum (FCS) or with various concentrations of SRC 2 (10%, 20%, 50% and 80%). Cell morphology is maintained in all assays. Increasing amounts of SCR2 increase the total number of cells per field (cell density).

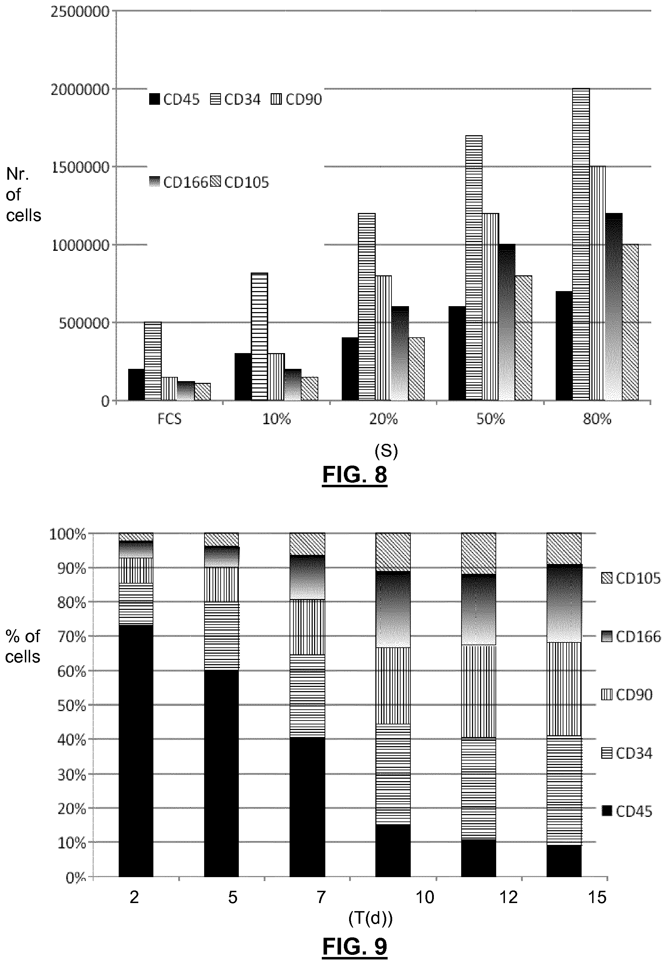

FIG. 8 is a diagram with the phenotype analysis of the bone marrow cells submitted to the conditions as indicated in FIGs. 6 and 7, that is, growth in a medium supplemented with FCS or with different concentrations of SRC 2 (10%, 20%, 50% and 80%). The phenotype is determined by the expression levels of several membrane antigens or markers for each of the assayed conditions (X-axis). The Y-axis shows the number of cells of each type. CD45: leukocyte marker; hematopoietic marker CD34, mesenchyme lineage marker CD90, CD166 stromal lineage marker; endothelial lineage marker CD105.
FIG. 9 represents the kinetics evolution of the different cell populations in cultures of bone marrow cells along the time (X-axis, T in days (d)). CD45: marker leukocyte; hematopoietic marker CD34, mesenchyme lineage marker CD90, CD166 stromal lineage marker; endothelial lineage marker CD105.

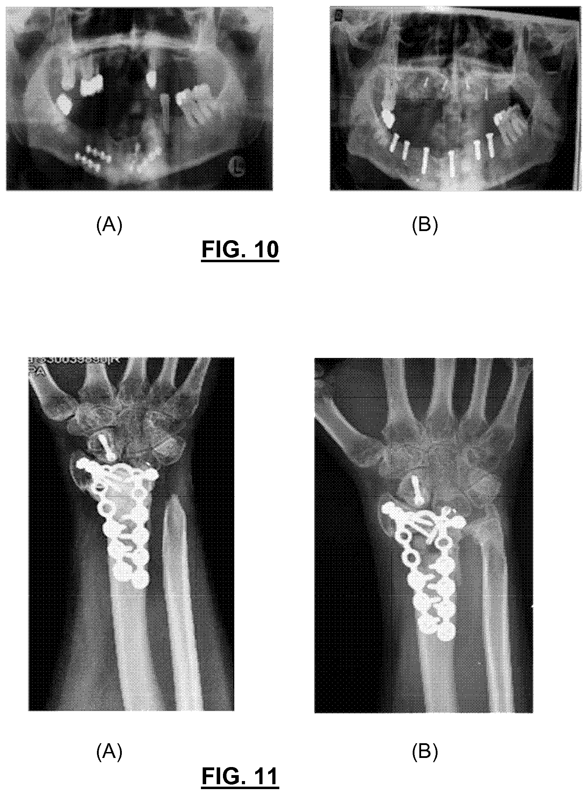

FIG. 10 is a magnetic resonance image (MRI) showing jaw bone regeneration. On the left side (Panel A) appears the mandibular state at the moment of surgical treatment, and on the right side (Panel B) the bone regeneration produced at 4 months after surgery and after some infiltrations or treatment with the serum according to the invention.
FIG. 11 is also an MRI image showing the scaphoid bone and ulna regeneration produced with a serum according to the invention. On the left (Panel A) the bone status at the time of surgery. On the right (Panel B) the bone formed at 6 months after surgery.

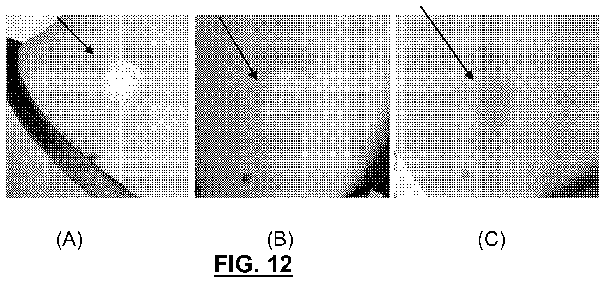

FIG. 12 shows photographs of the evolution of a hypertrophic scar before (Panel A) the injection of a serum according to the invention, and after the injection of two doses of the serum (Panel B) and 40 days after completing the treatment (Panel C). The arrow shows the position of the scar.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The following definitions are included for the purpose of understanding

In blood, the "serum" is the component that is neither a blood cell (serum does not contain white or red blood cells) nor a clotting factor; it is the blood plasma with the fibrinogens removed. Serum includes all proteins not used in blood clotting (coagulation) and all the electrolytes, antibodies, antigens, hormones, and any exogenous substances (e.g., drugs and microorganisms). Serum is obtained by blood centrifugation to remove cellular components. Anti-coagulated blood yields plasma containing fibrinogen and clotting factors. Coagulated blood (clotted blood) yields serum without fibrinogen, although some clotting factors may remain. Although associated with blood, there is also considered a serum, any fluid from a biological sample with leukocytes and/or platelets and fibrinogen that is let to form a clot. In the present description the expressions "serum comprising cytokines and coagulation factors" and "cytokine-rich serum" are used interchangeably and refer to a composition obtained from supernatants collected after submitting an isolated sample comprising platelets and/or leukocytes to a specified gravity force. This gravity force can be applied by several means including, for example, centrifugation of the sample.

A "coagulation factor" is a protein involved in the coagulation cascade. The coagulation factors are generally serine proteases (enzymes), which act by cleaving downstream proteins, although there are some exceptions (FVIII and FV are glycoproteins, and Factor XIII is a transglutaminase). Examples of coagulation factors are, Factor XII (Hageman Factor), Factor XI (plasma thromboplastin), Factor X, Factor IX (Christmas Factor), Factor V, Factor XIII (fibrin stabilising factor), Factor VII, Tissue factor (TF), prothrombin, thrombin, and fibrinogen. As indicated, the coagulation factors are involved in the coagulation cascade, which has two pathways which lead to fibrin formation. These are the contact activation pathway (also known as the intrinsic pathway), and the tissue factor pathway (also known as the extrinsic pathway). The pathways are a series of reactions, in which a zymogen (inactive enzyme precursor) of a serine protease and its glycoprotein cofactor are activated to become active components that then catalyze the next reaction in the cascade, ultimately resulting in cross-linked fibrin. Coagulation factors are generally indicated by Roman numerals, with a lowercase an appended to indicate an active form.

The term "cytokine" in the present invention, relates to any biological molecule signaling between cells. These molecules are produced by the cells themselves, and include, for example peptides, proteins, glycoproteins, mucoproteins, hormones, growth factors or enzymes having activity or being related to immune processes, inflammatory processes and reparative or regenerative processes in general. Cytokines (Greek cyto-, cell; and -kinos, movement) is the name used to describe a diverse group of soluble proteins, peptides, or glycoproteins which act as humoral regulators or signalling molecules at nano- to- picomolar concentrations and help in cell signalling. The term "cytokine" encompasses a large and diverse family of regulators produced throughout the body by cells of diverse embryological origin. The term "cytokine" has been used to refer to the immunomodulating agents, such as interleukins and interferons. They are regulators of host responses to infection, immune responses, inflammation, and trauma. Some of them are pro-inflammatory, these are necessary to initiate an inflammatory response necessary to recruit granulocytes, and later on, lymphocytes, to fight disease. Excessive inflammation, however, is sometimes the pathogenicity of certain diseases. Other cytokines are anti-inflammatory and serve to reduce inflammation and promote healing once the injury/infection/foreign body has been destroyed.

The term "Growth factor" is sometimes used interchangeably among scientists with the term cytokine. Historically, cytokines were associated with hematopoietic (blood forming) cells and immune system cells (e.g., lymphocytes and tissue cells from spleen, thymus, and lymph nodes). For the circulatory system and bone marrow in which cells can occur in a liquid suspension and not bound up in solid tissue, it makes sense for them to communicate by soluble, circulating protein molecules. However, as different lines of research converged, it became clear that some of the same signalling proteins the hematopoietic and immune systems used were also being used by all sorts of other cells and tissues, during development and in the mature organism. While growth factor implies a positive effect on cell division, cytokine is a neutral term with respect to whether a molecule affects proliferation. While some cytokines can be growth factors, such as G-CSF and GM-CSF, others have an inhibitory effect on cell growth or proliferation. Some cytokines, such as Fas ligand, are used as "death" signals; they cause target cells to undergo programmed cell death or apoptosis. Thus, in the sense of the present invention the term "growth factor" encompasses all other molecules, such as signalling proteins, peptides, glycoproteins, mucoproteins, or hormones that are not considered cytokines but that exert a signaling function between cells.

By "gravity force" is to be understood that force that mutually experience two bodies having a specified mass. Due to its mass, a body generates a gravitational field. A gravitational field may be also applied externally to a body by several means. One of these means include centrifugation that, due to the centrifugal acceleration impose to the mass a similar effect than gravity.

The term "centrifugation", relates to a method by which solids of different densities are separated from liquids through a rotating force, which applies to the mixture a centrifugal force greater than that of the gravity. Solids and the particles of greater density are then sedimented. Examples of centrifugation include, without limitation:
- Differential centrifugation: Based on the difference in the density of the molecules. This difference should be large enough to be observed by centrifuging. Particles having similar densities sediment together. This method is not specific, so it is used as a preparative centrifugation for separating components in the mixture (e.g., to remove nuclei and mitochondria membrane) but is not useful to separate molecules.
- Isopycnic centrifugation: Based on the fact that particles with the same sedimentation coefficient are separated by means of different density media. It is generally used for DNA separation.
- Zonal centrifugation: The particles are separated by the difference in the settling rate due to the difference in their mass. The sample is placed on top of a preformed density gradient. By centrifugal force the particles settle at different rates through the density gradient according to their mass. It has to be taken into account the centrifugation time because, if exceeded, all molecules could settle on the bottom of the test tube.
- Ultracentrifugation: Lets study the settling characteristics of subcellular structures (lysosomes, ribosomes and microsomes) and biomolecules.

The term " platelets" as used herein, refers to small cell units (2-3 microns in diameter), oval and without nucleus generated under normal conditions in the bone marrow from the cytoplasm fragmentation of megakaryocytes. Platelets circulate in the blood of all mammals and are involved in hemostasis, participating in the formation of blood clots. Platelets release a great number of growth factors and/or cytokines including the platelet-derived growth factor (PDGF, also abbreviated PDGFAA), a potent chemotactic agent and the transforming growth factor beta (TGF- β1), which stimulates extracellular matrix formation. Depending on certain conditions, the platelets also synthesize many other essential molecules of the inflammatory process and of the reparative and regenerative process of tissues. These growth factors or molecules have shown to play a significant role in the regeneration and repair of connective tissue. Other proteins and growth factors produced by platelets and inflammatory processes associated with, reparative and regenerative include basic fibroblast growth factor (BFGF), insulin-like growth factor-1 (IGF -1), insulin-like growth factor-2 (IGF -2), epithelial growth factor (EGF), hepatocyte growth factor (HGF), the vascular endothelial growth factor (VEGF), interleukin 8, the keratinocyte growth factor, connective tissue growth factor. Local application of all these growth factors, molecules and proteins produced by platelets participates and accelerates the healing process from different injuries. Platelets also release large amounts of thrombin and adenosine diphosphate ADP, calcium, thromboxane A2, PF4, PAI-1, fibrinogen, fibronectin, thrombomodulin, FV , FXIII, RANTES, thrombospondin, WWF PF-3, serotonin, hydrolytic enzymes, etc.

The term "leukocyte" or "white cells", used interchangeably herein, refers to blood cells that are part of cellular effectors of the immune system. They are cells with migratory capacity using the blood as a vehicle for accessing different parts of the body. Leukocytes are responsible for destroying infectious agents and infected cells. They also secrete cytokines, growth factors and protective substances, such as antibodies, that fight infections. They also activate biological functions in other cells. Based on microscopic features of their cytoplasm (staining) and of its nucleus (morphology), they are divided into:
- Granulocytes and polymorphonuclear cells (neutrophils, basophils and eosinophils) and have a polymorph nucleus and numerous granules in the cytoplasm, with differential staining according to cell types, and
- Agranulocytes or monomorphonuclear cells (lymphocytes and monocytes): they lack of cytoplasm granules and have a round nucleus.

The term "equivalent amounts of other soluble calcium source" in the present invention, relates to such amounts of calcium sources that allow the formation of ionized calcium in an amount similar to that indicated in the method. Soluble calcium sources are, for example, without limitation, calcium acetate, calcium carbonate, calcium glubionate, calcium gluconate, calcium hydroxide, calcium nitrate, calcium sulfonate, calcium phosphate, or other sources known to expert in the field. Changing sources of calcium and calculating equivalent amounts is simple and easily performed by a skilled artisan.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "effective amount" as used herein, means an amount of an active agent high enough to deliver the desired benefit (either the treatment or prevention of the illness), but low enough to avoid serious side effects within the scope of medical judgment.

The term "fibrinogen" in the present invention relates to a soluble protein from the blood plasma which is synthesized in the liver. It is responsible together with thrombin of fibrin formation in blood clots. When a wound is present, the coagulation cascade eventually transforms fibrinogen bound to thrombin to fibrin.

The term "thrombin" in the present invention refers to a glycoprotein enzyme which is not a normal constituent of the circulating blood, but is generated from prothrombin and factor II in the presence of calcium ions and by a reaction catalyzed by activated factor Xa, in the final step of the two converging cascades of reactions, the intrinsic pathway and the extrinsic pathway of coagulation. Thrombin activates fibrinogen degradation to fibrin monomers, besides activating factor XIII or fibrin-stabilizing factor. Other important biological functions of thrombin are noted: 1) the activation of the platelets so that they release the components (cytokines) of their α granules and dense granules; 2) promotion of the synthesis, release, and protein expression of endothelial cells (angiogenesis); and 3) chemotaxis potent induction of neutrophils (inflammatory factors) and macrophages (cytokine production).

The term "fibrin" as used herein, refers to a fibrillar protein with the ability to form three dimensional networks. This protein plays an important role in the coagulation process in view of its properties. It has the structure of a pole with three globular areas. It has the ability to create aggregates with other molecules forming a soft clot. Not normally found in the blood, it is formed from circulating fibrinogen, which by the action of the enzyme called thrombin is converted to fibrin, which has coagulant effects.

The term "fibrin gel composition" refers to the rigid and elastic gel (semi-solid composition) formed as intermediate layer when, due to centrifugation of a composition comprising platelets and/or leukocytes and fibrinogen (e.g. blood), activation of the coagulation cascade and synthesis of thrombin takes place for finally inducing fibrin formation. The fibrin forms next the three dimensional networks comprising, enmeshed, a serum (liquid) comprising cytokines, platelets and/or leukocytes in-between the fibrin fibers constituting the network or matrix. The fibrin gel can also be named fibrin matrix or fibrin scaffold, used herewith exchangeable as synonymous.

The term "sample" in the present invention, includes blood, bone marrow, umbilical cord blood, adipose tissue, placenta, menses or any substance from a subject, obtained by any method known by a skilled in the art which serves to carry out any of the methods provided by the present invention. That is, a sample that comprises at least platelets and leukocytes.

The term "blood", as used herein, refers to a tissue fluid flowing through capillaries, veins and arteries of all vertebrates. Its characteristic red color is due to the presence of the red pigment content of erythrocytes. It is a specialized type of connective tissue with a liquid colloidal matrix and complex constitution. Indeed, blood comprises a solid phase formed by cells (including leukocytes, erythrocytes and platelets) and a liquid phase, represented by plasma. Its main function is the logistic distribution and systemic integration, whose containment in vessels (vascular space) supports its distribution (blood circulation) to most of the body. The term "peripheral blood" relates to the distant circulating blood volume of the heart, that is, blood flowing through (along) the body of a subject.

The term "erythrocytes" or "red blood cells" or "RBC", in the sense used in this description, relates to blood corpuscles will carry the oxygen and carbon dioxide in the blood. Corpuscles are known for lacking nucleus and organelles (only in mammals), so cannot be considered strictly cells. They contain some enzymatic pathways and their cytoplasm is occupied almost entirely by hemoglobin, a protein responsible for transporting oxygen. In the plasma membrane of the erythrocytes are glycoproteins (CDs) that define the different blood groups and other cell identifiers. Erythrocytes are disc-shaped, biconcave, depressed in the center; this increases the effective surface area of the membrane. Mature RBCs lack a nucleus, because he is evicted in the last stage of maturation in the bone marrow before entering the bloodstream.

The term "subject" as used herein, refers to any animal with blood circulation, preferably a mammal, more preferably a primate, and still more preferably, a human being, male or female, of any age or race. In addition, the subject may be a health subject or a subject diagnosed of any pathology or disease.

The term "anticoagulant" in the sense used in this description, refers to a endogenous or exogenous substance that interferes with or inhibits blood clotting. Non-limiting examples of anticoagulants are the activators of antithrombin III and heparin, low molecular weight ardeparin, certoparin, nadroparin, logiparin, parnaparin, reviparin and tedelparina and recombinant antithrombin III, tissue factor inhibitor (TF)-Factor Vila such as TF mutants, anti-TF antibodies, inhibitors of factor VII, thrombin inhibitors such as antistatin factor Xa inhibitors, TAP (tick anticoagulant peptide), DX9065, lefaxin, fondaparinux, terostatin, YM-75466 and ZK-807,834, antibodies againts factor IXa and Xa, activators of protein C pathway and selective inhibitors of thrombin as argatroban, bivalirudin, efegatran, H376/95, hirugen, inogatran, melagatran, napsagatrán, UK-156406 and ximelagatran, and chemical compounds that capture calcium ions, such as ethylenediaminetetraacetic acid (EDTA), citrate (usually sodium citrate) and oxalate.

The term "platelet rich plasma" or "PRP" in the present invention relates to a plasma in the presence of platelets. The PRP may be prepared by any conventional method known to those skilled in the art, such as, the method disclosed in US 20050252867. An example of easy preparation of PRP is a method comprising:
I) removing a blood sample from a subject in the presence of sodium citrate as an anticoagulant,
II) centrifuging the sample at 150 g for 10 minutes, and
III) to obtain the supernatant where the supernatant is the PRP.

The term "platelet-poor plasma" or "PPP" in the present invention relates to a plasma fraction remaining after separating and obtaining a platelet concentrate or "cPLT".

The term "platelet concentrate" or "cPLT" in the present invention relates to a preparation of platelets with a concentration of platelets which is at least three times greater than the mean baseline in blood. It is the remaining fraction of plasma after obtaining a "platelet poor plasma" or "PPP".

The term "plasma concentrate" and/or "serum concentrate" in the present invention relates to a plasma or serum with a higher concentration of protein. An example of obtaining a plasma or serum concentrated can be for example, without limitation, the method comprising:
I. centrifuge one plasma or serum at a speed of between 5000 g and 6500 g for 5 to 10 minutes,
II. removal of the supernatant and re-suspension the pellet in the remaining supernatant.

The term "plasma" or "blood plasma" in the present invention relates to the liquid fraction of blood. It is composed of 90% water, 7% protein, and the remaining 3% of fat, glucose, vitamins, hormones, oxygen, carbon dioxide and nitrogen, as well as metabolic waste products such as uric acid. It is the major component of blood, representing approximately 55% of total blood volume, while the remaining 45% corresponds to the formed elements. Blood plasma viscosity is 1.5 times that of water. Major plasma proteins are the following: Albumin, which also participates in the transport of lipids; Globulins, which are mainly related to the body's defense mechanisms (e.g. immunoglobulins), or with iron transport (transferrin); Fibrinogen, a protein that is essential for blood clotting; Prothrombin, protein involved in the clotting process; and Lipoproteins and low density lipoproteins (LDL and HDL, respectively) which are involved in cholesterol transport. The set of plasma proteins, also called "plasma factors", perform the functions, among others, of:
- Maintain plasma volume and blood volume,
- Buffering and maintaining the pH stability of blood,
- Participate in the viscosity of the blood, and hence, contribute minimally to peripheral vascular resistance and vascular pressure (blood pressure), and
- Intervene in the electrochemical equilibrium ion concentration (called Donnan effect).

The term "leukocyte-rich plasma" or "plasma leukocyte concentrate" (PRL) in the present invention refers to a blood sample which has been treated to eliminate all the cell types different of leukocytes, and which has a leukocyte concentration higher than the original in the blood sample. An example of a method for obtaining leukocyte rich plasma may be for example, a method comprising:
I) A whole blood sample collected and separated by centrifugation into a packed red blood cells and plasma containing white blood cells (buffy coat).
II) In a second step the fraction of plasma containing white blood cells (buffy coat),is centrifuged to separated pour plasma or supernatant from white blood cells (buffy coat).
III) Once removed the pour plasma or supernatant the remaining sample (pellet) are leucocytes cells (buffy coat) or leukocyte-rich plasma, the final volume can be adjusted

The term "regeneration of tissue" or "tissue regeneration" in the present invention, relates to the total or partial process of renewal, restoration and growth of cells, tissues and organs after they have been disrupted or damaged. Tissue regeneration refers to the biochemical, morphological and functional processes conceived for repairing and maintaining the integrity of the physiological and morphological state thereof. Repair and regeneration are synonymous in many cases, even in the case of the regeneration a greater emphasis is done on a more complete recovery, both morphologically and physiologically. Indeed a "tissue reparative agent" is an agent that allows the tissue to recover its normal function although not new tissue (or cells) has been created.

The term "cell proliferative agent" relates to any compound or composition that favours cell division. By "cell differentiating" agent is to be understood any compound or composition that favours immature cells to become mature cells with specified and differentiated functions.

As above indicated, the invention discloses a method for preparing serums comprising cytokines and coagulation factors, the method comprising the steps of (a) submitting an isolated biological sample comprising platelets and/or leukocytes to a gravity force comprised from 100 g to 200 g to obtain a first clot and a supernatant; (b) submitting the clot or, alternatively, the clot and supernatant of step (a) to a gravity force comprised from 400 g to 2500 g to obtain a supernatant SN1; wherein the gravity force is discontinuously applied and carried out by means of:
(i) centrifuging the clot of step (a), or alternatively the clot and supernatant, at a gravity force comprised from 400 g to 500 g;
(ii) recovering the supernatant of step (i), which constitutes partially the supernatant SN1ii;
(iii) centrifuging the remaining clot at a gravity force comprised from 1500 g to 2500 g and recovering the supernatant of step (iii), which constitutes partially the supernatant SN1iii of step (b), constituting the supernatants of steps (ii) and (iii) the entire supernatant SN1; and (c) recovering said supernatant SN1 of step (b), which is a serum comprising cytokines and coagulation factors,
wherein the gravity force is applied by a process selected from the group consisting of centrifugation and physical-suction.

Thus, this method allows the obtention of a serum comprising cytokines and coagulation factors herewith called supernatant SN1.

In a particular embodiment of this method, prior to step (b) the clot or, alternatively, the clot and supernatant of step (a) is submitted to a gravity force comprised from 1 g to 10 g. This particularity of the method allows stabilizing the clot, which later will be able to produce many cytokines. Thus, although not essential for the method, if the gravity force is lowered between steps (a) and (b), or if a resting period is permitted, improved results are achieved (more concentrations of cytokines and of coagulation factors).

Also it is particularly beneficial if step (a) is performed not later than 5 minutes after isolation of the sample. By this way, those coagulation factors of the sample are in the especially good conditions to perform its role. That is, protein denaturisation is minimized.

In another particular embodiment of the first aspect of the invention, there is performed an additional step (d) after step (c). In this step (d) it is performed the adjustment of the ionic calcium to a final concentration from 1.0 µmol/ml to 50.0 µmol/ml and of the pH of the final solution to a pH comprised from 7.0 to 7.6, by adding an ionic calcium source to the recovered supernatant and, optionally, a pH adjusting agent. The pH adjusting agent can be ionic calcium source itself if the accompanying anion of calcium is an anion of a physiologically acceptable buffer (see below).

The method of the invention may thus include a final step in which is added a source of calcium to allow the formation of ionized calcium in the sample and thereby to adjust the pH from 7.2 to 7.6. This formation of ionized calcium is essential for many cellular and biological processes. Calcium is an important intracellular second messenger (transport in the cytoplasm) and biological regulator required for normal cell function, is required by many proteins, enzymes and molecules for activity and cell signaling.

Calcium-pH relationship is linear from a pH value between 7.20 and 7.60 with normal levels of total proteins. Moreover adjustment of pH is important to achieve an homeostatic environment, in which a proper cellular activity and signaling can takes place. Suitable ionic calcium sources include pharmaceutically acceptable salts of calcium, such as chloride calcium, calcium acetate, calcium carbonate, calcium glubionate, calcium gluconate, calcium hydroxide, calcium nitrate, calcium sulfonate, and calcium phosphate. This calcium sources are all suitable pH buffers. Other pH buffers may also be used, meanwhile ionic force of the sample is not jeopardized and no toxic for the cells compounds are introduced.

When the biological sample is in particular blood, this step (d) is not necessary if blood is in the considered normal physiological conditions, which means a ionic calcium concentration from 1.0 µmol/ml to 50.0 µmol/ml; and a pH from 7.0 to 7.6. Anyway, if the serum has to be used, for example, to stimulate other cells or to further activate compositions with platelets and/or leukocytes, it is preferred to adjust pH and calcium concentration. Adjustment may also be performed if the serum has to be stored for a time.

As above indicated the gravity force is in particular applied by centrifugation. Centrifugation imposes an effect equivalent to gravitation by means of a rotation force.

In another particular embodiment, when the gravity force of a specified magnitude is applied in a step, independently of being applied by centrifugation or not, the time of this application is comprised from 2 to 30 minutes. In another more particular embodiment, the gravity force is applied from 5 to 15 minutes and most in particular for 10 minutes. Depending on this time, a different cytokine profile is obtained as well as a different concentration of each of the cytokines. In any case, however, the serum is enriched in the cytokines and coagulation factors, since the gravity force activates the cells of the starting material (isolated biological sample).

In a particular embodiment, the centrifugation or gravity force of step (i) is 450 g; and that of step (iii) is 1500 g.

The centrifugation of step (iii) imply a gravity force (or centrifugation) sufficient to break the bonds and to produce fibrin degradation in the precipitate formed in step (i). Furthermore, gravity force must be sufficient to produce a release of the cells entrapped in the fibrin clot present in this precipitate. By this way the cells can be activated to trigger cytokine production without giving rise to the lysis thereof.

Thus, in the method disclosed herein, the steps allow the application of a specified pattern of gravity force, which in particular cases is a centrifugal force. Firstly, the submission to a gravity force from 100 g to 200 g allows formation of the clot (containing platelets and/or leukocytes embedded in a fibrin matrix) and a mild activation of the same. That is, promoting initiating the interaction between cells and the synthesis or release of primary cytokines, basically, from the platelets of the biological sample. Then, by means of a higher gravity force (from 400 g to 2500 g), the clot is vigorously activated, in such a way that platelets as well as leukocytes of the sample release other cytokines to the supernatant. These other cytokines are mostly those appearing late when coagulation pathway is activated. They also include the molecules that are needed later in the signalling between the cells, when it is needed to modulate other late events in the processes wherein these types of cells are involved.

In a particular embodiment, the gravity force of step (a) is of 150 g, applied by centrifugation of the sample or by other means (i.e. suction).

Furthermore, the method is in particular carried out at a temperature comprised from 18 °C to 40 °C. In particular at 37 °C, thus mimicking the physiological conditions. The control of the temperature assures that no degradation of the obtained product takes place.

In another particular embodiment of the method of the invention, between steps (a) and (b), or between the successive sub-steps (i) and (iii), there are carried out resting periods sufficient to form the clot. This allow completing the coagulation cycle (activation and platelet degranulation/release of cytokines and growth factors from α granules and dense granules), and further to initiate fibrinolysis. Therefore, this resting step should be in particular comprised from 1 to 60 minutes, more particularly from 1 to 30 minutes, and even more particularly of 5 minutes.

Another particular and specific embodiment of the method of the invention relates to a method for preparing cytokine-rich serums, said serums comprising cytokines and growth factors, the method comprising:
- Centrifuging a previously isolated biological sample obtained from a subject in the absence of anticoagulants and containing a final 15 µmol/ml of a calcium source in the sample, such as CaCl₂ (from a solution at 10 % w/w, thus comprising 2.5 mg / ml in the calcium source), or other sources of ionized calcium;
- allow the precipitate for 1 to 60 minutes at a temperature comprised between 18 and 40 ° C.
- centrifuging the sample of previous step,
- extracting the sample supernatant from previous step,
- centrifuging the resting precipitate from the centrifugation step,
- extracting the supernatant from this second step of centrifugation,
- mixing the supernatants or serums obtained in previous steps,
- adding a source of ionized calcium to a final concentration of from 1.1 to 1.4 mol / ml in the sample sample (4.5-5.6 mg / dL) and adjust the pH between 7.2 and 7.6.

Yet in another particular embodiment of this first aspect of the invention, the isolated biological sample is selected from blood, bone marrow, umbilical cord blood, adipose tissue, and placenta. In a preferred embodiment, the sample is isolated blood. In a more preferred embodiment blood is peripheral blood. The main issue is that the isolated sample contains fibrinogen, platelets and/or leukocytes. So that, they can form a clot (or precipitate) containing the platelets and/or the leukocytes in a fibrin matrix.

The method of the invention may further comprise a step in which almost of the erythrocytes are completely eliminated. Erythrocytes (red blood cells), besides of its contribution as oxygen transporters, they do not exert a significant role in the regenerative process. This extraction of red blood cells can be performed by means of several techniques known in the art, such as but not limited to, layer suction or gravity sedimentation or centrifugation. Indeed, erythrocytes sediment at the bottom of the tube when centrifugation is applied to a sample. They can be easy removed.

Thus in a preferred embodiment of the method of the invention, the method further comprises extracting the fraction of red blood cells after step (a), or (b) or, after an optional resting step.

On the other side, yet a low concentration of red blood cells can be beneficial. So that, if an amount of at least 2 % w/w remains in the sample or in any of the clots and supernatants obtained along the performance of the method, the red blood cells can act as oxygen source (due to hemoglobin) and help in the cell metabolism.

The method of the invention may additionally contain other steps to enrich certain cytokines in the final product depending on the requirements of each case, and depending on the utility of the serum. These additional elements represent the complement of one of the products (serums) obtainable by the method of the invention.

Thus, the method may further comprise the steps of:
- mixing the supernatant SN1 of step (c) with a composition comprising fibrinogen, platelets and/or leukocytes selected from the group consisting of a platelet-rich plasma, a platelet concentrate, a leukocyte-rich plasma, a leukocyte concentrate, a platelet-poor plasma, a plasma concentrate and mixtures or combinations thereof;
- adding to the mixture of previous step an ionic calcium source to adjust the ionic calcium to a final concentration comprised from 1.0 µmol/ml to 50.0 µmol/ml to obtain a second clot, which is a fibrin gel composition comprising platelets and/or leukocytes embedded in a fibrin matrix, said gel comprising also a serum with cytokines and coagulation factors in liquid form disposed in-between the fibrin matrix.

Therefore, it is also a part of the invention a method for preparing a fibrin gel composition comprising platelets and/or leukocytes embedded in a fibrin matrix, said gel comprising also a serum with cytokines and coagulation factors in liquid form disposed in-between the fibrin matrix, the method comprising the following steps:
(a) Submitting an isolated biological sample comprising platelets and leukocytes to a gravity force comprised from 100 g to 200 g to obtain a clot and supernatant;
(b) Submitting only the clot or, alternatively, the clot and supernatant of step (a) to a gravity force comprised from 400 g to 2500 g to obtain a supernatant SN1 wherein the gravity force is discontinuously applied and carried out by means of:
   (i) centrifuging the clot of step (a), or alternatively the clot and supernatant, at a gravity force comprised from 400 g to 500 g;
   (ii) recovering the supernatant of step (i), which constitutes partially the supernatant SN1ii;
   (iii) centrifuging the remaining clot at a gravity force comprised from 1500 g to 2500 g and recovering the supernatant of step (iii), which constitutes partially the supernatant SN1iii of step (b), constituting the supernatants of steps (ii) and (iii) the entire supernatant SN1;
(c) Recovering said supernatant SN1 of step (b), which is a serum comprising cytokines and coagulation factors;
(d) mixing the supernatant SN1 of step (c) with a composition comprising fibrinogen, platelets and/or leukocytes selected from the group consisting of a platelet-rich plasma, a platelet concentrate, a leukocyte-rich plasma, a leukocyte concentrate, a platelet-poor plasma, a plasma concentrate and mixtures or combinations thereof; and
(e) adding to the mixture of previous step an ionic calcium source to adjust the ionic calcium to a final concentration comprised from 1.0 µmol/ml to 50.0 µmol/ml;
wherein the gravity force is applied by a process selected from the group consisting of centrifugation and physical-suction.

Yet is to be considered an object of the invention this fibrin gel composition comprising the platelets and/or leukocytes embedded in a fibrin matrix, and also comprising a serum with cytokines and coagulation factors in liquid form disposed in-between the fibrin matrix. This semi-solid (i.e. gel) product is indeed a rigid and elastic gel composition comprising the serum defined by supernatant SN1, together with a source of cells and ionic calcium.

In a particular embodiment of this previous method, the fibrin gel composition with the platelets and/or leukocytes embedded in a fibrin matrix, and comprising a serum with cytokines and coagulation factors in liquid form disposed in-between the fibrin matrix, is submitted to physical and/or chemical and/or biochemical means to break the fibrin matrix by these means, and so to obtain a supernatant SN2. This supernatant SN2 is recovered and is also a serum comprising cytokines and coagulation factors.

Thus, it is also part of the invention a method for preparing a serum comprising cytokines and coagulation factors, named SN2, the method comprising the following steps:
(a) Submitting an isolated biological sample comprising platelets and/or leukocytes to a gravity force comprised from 100 g to 200 g to obtain a first clot and supernatant;
(b) Submitting only the clot or, alternatively, the clot and supernatant of step (a) to a gravity force comprised from 400 g to 2500 g to obtain a supernatant SN1; wherein the gravity force is discontinuously applied and carried out by means of:
   (i) centrifuging the clot of step (a), or alternatively the clot and supernatant, at a gravity force comprised from 400 g to 500 g;
   (ii) recovering the supernatant of step (i), which constitutes partially the supernatant SN1ii;
   (iii) centrifuging the remaining clot at a gravity force comprised from 1500 g to 2500 g and recovering the supernatant of step (iii), which constitutes partially the supernatant SN1iii of step (b), constituting the supernatants of steps (ii) and (iii) the entire supernatant SN1;
(c) Recovering said supernatant SN1 of step (b), which is a serum comprising cytokines and coagulation factors;
(d) mixing the supernatant SN1 of step (c) with a composition comprising fibrinogen, platelets and/or leukocytes selected from the group consisting of a platelet-rich plasma, a platelet concentrate, a leukocyte-rich plasma, a leukocyte concentrate, a platelet-poor plasma, a plasma concentrate and mixtures or combinations thereof;
(e) adding to the mixture of previous step an ionic calcium source to adjust the ionic calcium to a final concentration comprised from 1.0 µmol/ml to 50.0 µmol/ml to obtain a second clot, which is a fibrin gel composition with the platelets and/or leukocytes embedded in a fibrin matrix, and comprising a serum with cytokines and coagulation factors in liquid form disposed in-between the fibrin matrix;
(f) submitting the fibrin gel composition to physical and/or chemical and/or biochemical means to break the fibrin matrix by these means to obtain a supernatant SN2;and
(g) recovering the supernatant SN2;
wherein the gravity force in steps (a) and (b) is applied by a process selected from the group consisting of centrifugation and physical-suction.

This method allows the obtention of another generation of cytokine-rich serums (serums comprising cytokines and coagulation factors). These final serums, herewith also identified as supernatants SN2, confer unique characteristics and represent non-existing in nature compositions, because the levels and balance of cytokines and coagulation factors that can be obtained is higher. Thus, the final supernatants SN2 will have a therapeutic potential higher than the ones of the natural body systems.

These serums SN2 can also be defined by their composition, so that, they comprise cytokines (including growth factors) and coagulation factors selected from the group consisting of:
- Transforming growth factor beta (TGF-β 1), which concentration in the final serum is comprised from 100.0 ng/ml to 200.0 ng/ml;
- Insulin growth factor-1 (IGF-1), which concentration in the final serum is comprised from 200.0 ng/ml to 350.0 ng/ml
- Hepatocyte Growth Factor (HGF), which concentration in the final serum is comprised from 10.0 ng/ml to 50.0 ng/ml;
- Epidermal growth factor (EGF), which concentration in the final serum is comprised from 70.0 pg/ml to 200.0 pg/ml;
- Vascular Endothelial Growth Factor (VEGF), which concentration in the final serum is comprised from 200.0 pg/ml to 700.0 pg/ml;
- Interleukin 6 (IL-6), which concentration in the final serum is comprised from 3.0 pg/ml to 50.0 pg/ml;
- Interleukin 8 (IL-8), which concentration in the final serum is comprised from 2.0 pg/ml to 100.0 pg/ml;
- Interleukin 10 (IL-10), which concentration in the final serum is comprised from 5.0 pg/ml to 30.0 pg/ml;
- Derived Growth Factor P (PDGF), which concentration in the final serum is comprised from 40.0 ng/ml to 250.0 ng/ml;
- Factor VII, which concentration in the final serum is comprised from 200.0 U/dl to 350.0 U/dl, wherein U means enzymatic units;
- von Willebrand Factor, which concentration in the final serum is comprised from 120.0 U/dl to 300.0 U/dl; and mixtures thereof.

The serum SN2 is capable of inducing proliferation of bone marrow cells in such a way that, starting from a culture of bone marrow cells seeded in a Petri dish with a density comprised from 150 cells /cm² to 250 cells /cm², by addition of the serum in a percentage by weight comprised from 10 % to 30 % in the final culture, the amount of hematopoietic cells identified by the membrane marker CD34 is comprised from 1.0 x 10⁶ to 1.5 x 10⁶ measured 15 days after contacting the bone marrow cells with the serum. The culture medium supplemented with an amount of serum comprised from 10 % to 30 % w/w may be any of the known by the skilled man in which bone marrow cells can grow. An example is a Dulbecco's modified medium comprising glucose and having 4 mM L-Glutamine, and 0.05 units of penicillin and 0, 05 g of streptomycin.

The principle of this is that, the supernatant of step (c), which is a serum with cytokines and coagulation factors, acts as a source of thrombin (coagulation factor) and of a specified pattern of cytokines that activate the cellular elements of the composition comprising fibrinogen, platelets and/or leukocytes. In addition, the source of calcium added to promote clotting also acts further as chemical mediator or activator of the cells. As a result, the platelets and/or leukocytes synthesize and produce additional cytokines and coagulation factors. In a particular embodiment, the ionic calcium source is added in step (e) to a final concentration in the mixture of 24 µmol/ml. An example of source is a CaCl2 solution at 10% w/w (4mg/ml). However, any source of calcium would also be useful as above indicated.

In another particular embodiment, when the supernatant SN1 of step (c) is mixed with a composition comprising fibrinogen, platelets and/or leukocytes to finally obtain a clot (i.e. a fibrin gel composition), a gravity force comprised from 400g to 2500g is used as a physical means to break the fibrin matrix for obtaining and recovering again a supernatant SN2. In a particular embodiment, the physical means are centrifugation.

In another particular embodiment, the physical means to break the fibrin matrix is also a gravity force applied discontinuously by means of:
(i) centrifuging the fibrin gel composition with the platelets and/or leukocytes embedded in a fibrin matrix and comprising a serum with cytokines and coagulation factors in liquid form disposed in-between the fibrin matrix, at a gravity force comprised from 400 g to 500 g;
(ii) recovering the supernatant of step (i), which constitutes partially the supernatant SN2; and
(iii) centrifuging the remaining fibrin gel composition at a gravity force comprised from 1500 g to 2500 g and recovering the supernatant of step (iii), which constitutes partially the supernatant SN2, constituting the supernatants of steps (i) and (iii) the entire supernatant SN2, which is a serum comprising cytokines and coagulation factors.

On the alternative, the physical means to break the fibrin matrix is a gravity force applied as a continuous increasing gradient of gravity force from 400 g to 2500 g.

To be of adequate coagulation and adequate formation of additional cytokines, serum/supernatant of step (c) and the composition comprising fibrinogen and platelets and/or leukocytes are in particular mixed in appropriate proportions. Thus, in a particular embodiment the ratio of the serum of step (c) and the indicated composition is comprised from 10:1 to 1:1.

On the other hand, note that any clot (fibrin gel composition) formation is essential to trigger the production of cytokines properly. The speed of clot formation depends on the coagulation factors from the donor or isolated sample. However, if the resulting serum SN2 has to be finally administered to the subject (i.e. in an operating room, for example), the clot formation must be quick so that the process is carried out in short time. Therefore, in a more preferred embodiment, the clot or fibrin gel composition with the platelets and/or leukocytes embedded in a fibrin matrix and comprising a serum with cytokines and coagulation factors in liquid form disposed in-between the fibrin matrix is produced in a time less than a minute. The same applies with respect to the clot of step (a) if the supernatant SN1 (serum) is further used in the treatment of a subject in an operating room.

Fibrin matrix degradation can be produced by mechanical or by biochemical or biological methods, such as by centrifugation, agitation or addition of enzymes. However, the addition of foreign elements can cause contamination in the samples, or the inclusion of elements non compatible with the organism that finally will receive the serums for use in any treatment. Therefore, fibrin degradation is advantageously performed by physical means such as stirring or centrifugation. This agitation or centrifugation must be carried out in a sufficiently intensity to degrade the fibrin matrix without causing the deterioration of other significant factors in the final serum. Therefore, in another more preferred embodiment, fibrin degradation of step (f) is performed by centrifugation from 400 g to 2000 g.

In a particular and specific embodiment of the method of the invention when a composition comprising platelets and/or leukocytes and fibrinogen is added, the method comprises the steps of:
- mixing the supernatant or serum obtained in step (c) with a composition selected from a platelet rich plasma, a platelet concentrate, a concentrate rich plasma, a leukocyte concentrate, a platelet poor plasma, and a plasma concentrate,
- adding CaCl2 to the mixture of previous step to a concentration of CaCl2 24 µmol/ml 10% (4mg/ml) in the sample or equivalent amounts of other soluble calcium source,
- allow or wait to the formation of a clot in the mixture,
- degrade the fibrin matrix of the clot formed in previous step step by physical, biochemical, or biological means,
- to obtain the supernatant or serum samples derived from the combinations of previous steps, and
- adding ionized calcium to a final concentration comprised from 1.1 to 1.4 µmol/ml (4.5-5.6 mg / dL) and adjust the pH between 7.2 and 7.6.

All these variants of the method of the invention allow the production of serums, or of fibrin gel compositions, with high concentrations of cytokines and coagulation factors as will be exposed in the foregoing examples.

In another embodiment, the pharmaceutical compositions comprising the serums, or alternatively the fibrin gel compositions of the invention, are those in which besides any pharmaceutically acceptable carriers and/or excipients, comprise an ingredient selected from the group consisting of stem cells, cells of the immune system, a platelet-rich plasma, a platelet concentrate, a leukocyte-rich plasma, a leukocyte concentrate, a platelet-poor plasma, a plasma concentrate and mixtures or combinations thereof.

The activation of the cell component of these types of compositions can take place spontaneously or may be aided by the addition of a calcium source in the moment of use or at a proper time previous to the use of the compositions, as above exposed.

Serums obtainable by the method of the invention can be used independently or in conjunction with other elements known to the skilled person, for example, for preserving or maintaining the properties of the serum, or to supplement its activity. Compositions comprising these additional elements have the same utility as the serum of the invention. Examples of elements that can be included in these compositions are elements that enhance the activity of serum, or carriers that can function as a support or scaffold for better application of the serum. Therefore, another aspect of the invention relates to a composition, hereinafter composition of the invention comprising the serum of the invention. In a preferred embodiment, these compositions further comprise fibrin, fibrin sealant, Tachosil, FloSeal® Hemostatic Matrix, gelatin-based hemostatic agents, surgical hemostatic patches, hyaluronic acid, glycosaminoglycans, alginates, collagen, glycogen, glucosamine, glucuronic acid, amylose, dextrins, poly (alpha-hydroxy acid) s and copolyesters of lactic acid and glycolic acid (lactic), polyethylene glycol, hydroxyethyl starch and variants, chitin and chitosan, dextran, polyethylene glycol, and derivatives cyanoacrylates, polypropylene, PEG / dextran / L-DOPA related molecules liposomes, liposomes, liposome-hydrogel hybrid, bone morphogenic protein (BMP), growth factors, netrin-1, MMP7, bioactive matrix composed of active molecules, silicone, poly (DTE carbonate), Matrigel®, hydrogel microparticles, bioceramics, glutaraldehyde, chondroitin sulphate and / or pentosan polysulfate.

The serums, or alternatively the fibrin gel compositions, or alternatively the pharmaceutical compositions according to the invention are for use as a medicament. In a particular embodiment of this aspect of the invention, the serums, or the fibrin gel compositions or the pharmaceutical compositions comprising cytokines and coagulation factors, are for use in the treatment of a disease or pathology selected from the group consisting of an inflammatory pathology, a degenerative disease, a disease caused by ischemia, a vascular disease, an immunological pathological process, and trauma.

Examples of inflammatory pathologies include rheumatoid arthritis, which is also an immunological pathological process (autoimmune disease); arthritis and tendonitis. Degenerative diseases include Osteoporosis, Multiple sclerosis, and Muscular dystrophy. Vascular diseases include atherosclerosis, Peripheral vascular disease (PVD), and Renal Artery Disease. Immunological pathological processes include Systemic lupus erythematosus, Type 1 diabetes, and Guillain-Barre syndrome. Diseases caused by ischemia include Peripheral artery disease (PAD), stroke, and cardiopathies. These diseases are those which origin is due to a restriction in blood supply to tissues, causing a shortage of oxygen and glucose needed for cellular metabolism. Finally, trauma relates to injuries caused by accidents or surgical interventions.

This particular embodiment may also be formulated as the use of the serums as defined above, or of the fibrin gel compositions as defined above, or of the pharmaceutical compositions as defined above, for the manufacture of a medicament for the treatment or prevention of a disease or pathology selected from the group consisting of an inflammatory pathology, a degenerative disease, a disease caused by ischemia, a vascular disease, an immunological pathological process, and trauma. The present invention also relates to a method for the treatment or prevention of a disease or pathology selected from the group consisting of an inflammatory pathology, a degenerative disease, a disease caused by ischemia, a vascular disease, an immunological pathological process, and trauma, comprising administering a pharmaceutically effective amount of any of the serum as defined above, or of the fibrin gel composition together with pharmaceutically acceptable excipients or carriers, in a subject in need thereof, including a human.

In a particular embodiment, the serums of the invention or any composition comprising them (for example the fibrin gel compositions), are for use in any of the above-listed pathologies, wherein the disease or pathology is in a body region selected from the group consisting of the locomotive apparatus, in the osteoarticular system, in the skin, in the mucoses, in the vascular system, in the cardiovascular system, in the reproduction system, in the ocular system, in the gastrointestinal system, in the earing system, in the central nervous system, in the peripheral nervous system, and in the immune system.

Indeed, the serums, fibrin gel composition and/or pharmaceutical compositions are, especially, for the treatment of wounds. Using these products according to the invention (serums, gels, and pharmaceutical compositions), injured tissues are quickly repaired or regenerated.

Wounds are defined as lesions in a body area of a human or animal. Although the mostly known wounds are those in the surface skin, internal organ surfaces can also be injured for example by surgery or by rupture due to a trauma. With the serums of the invention any wound can be healed, being external or internal thanks to the faculty of providing in the wound or nearby all those components for an accelerated healing.

Thus, in another particular embodiment the serums and compositions (fibrin gel compositions and pharmaceutical compositions) of the invention are for use as organ and/or tissue reparative and regenerative agents.

Also in another particular embodiment, the serums, as well as the fibrin gel compositions (or any pharmaceutical composition comprising any of them) can be used in the treatment of scars, such as epidermal scars, since the cytokines present in the serums or compositions in high amounts are, in addition, able to promote right cicatrisation and the reversion of any pathological (i.e. keloides), or non-aesthetical scar.

Yet in another particular embodiment, the serums and compositions comprising them are for use as agents able to activate and/or promote angiogenesis and/or vasculogenesis.

The serums of the invention, or the fibrin gel compositions, are for use in the modulation and regulation of inflammatory diseases, immunological diseases, modulation and regulation of tissue regeneration. In the sense of the invention modulation and regulation means affecting the balance and amounts of the molecules and cells involved in inflammatory processes that finally lead to the reparation o recovery of injured tissues due to any etiology (trauma, immunological disease, etc.).

The term tissue repair or recovery means that the tissue or organ acquires those lost due to injury biochemical and physiological functions, and the morphological features.

The invention also encompasses a *"ex vivo"* organ perfusion liquid composition comprising, as an ingredient, the cytokine-rich serum (SN1 or SN2) as defined above or any liquid composition comprising this cytokine-rich serum.

Notably, the serums (SN1, SN2) of the invention have utility to increase the compatibility of perfused organs being embedded in the serum of the invention prior to implantation in a patient. This utility is further enhanced when the serum obtainable by the method of the invention comes from the transplant organ recipient. Cytokines regulate both inflammatory responses and specific immune response. Inflammatory changes occur in the transplantation of organs as a result of tissue trauma and of ischemia / reperfusion injury in the organ donor and at the time of the transplant operation. In all, cytokines play a major role in mediating these changes. These inflammatory changes may not only affect graft function, but also influence the immunogenicity of graft rejection and vulnerability. Cytokines orchestrate the specific immune response induced by organ transplantation.

The identification and measurement of circulating cytokines within biological fluids produced in organ transplantation can aid in the diagnosis of graft rejection and other complications after the transplant. Organ ischemia and reperfusion injury partly determine short-and long-term graft survival. The organ preservation conditions seem to play a decisive role. Therefore, another aspect of the invention relates to the use of a serum obtainable by the method of the invention for maintaining perfusion of organs or *"ex vivo".*

The serums SN1 and SN2 obtainable by the methods of the invention, present the optimal characteristics to be used as mediums in which maintain or reproduce differentiating cells, as it comprises the elements required for this function, most of the cytokines and growth factors. Therefore, another aspect of the invention relates to the use of a serum obtainable by the method of the invention as *in vitro* or *ex vivo* cell culture medium.

Another aspect of the invention is a cytokine-rich serum SN1 or SN2 as defined above or any composition comprising it, for use as an *in vitro,* and/or *ex vivo* and/or *in vivo* cell proliferative and/or cell differentiating agent.

The method of the invention can be performed manually by a technician, or may be carried out fully automated. This can be done using a medical device comprising in an integrated manner many technologies, such as mechanical, electrical, electromechanical, electronics, hydraulics, optics (scanner, laser), waves, and radiofrequency. The physical elements of the device include pumps, centrifuges, sensors, detectors, transducers and/or control and monitoring software. The method can also be performed employing single-use medical devices that can go together or separately and connected to power equipment which is single-use, sterile and non-pyrogenic. It is composed of plastics, polymers, derivatives thereof or the like. The main reason for using this device is the infection control, and to avoid transmitting infectious agents to operators and patients. Therefore, another aspect of the invention relates to an electro-medical device and / or disposable for performing the method of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

The following specific examples provided in this patent illustrate the nature of the present invention. These examples are included for illustrative purposes only and should not be interpreted as limitations to the invention claimed herein. Therefore, the examples described below illustrate the invention without limiting the scope thereof. Below the invention will be illustrated by tests performed by the inventors, which shows the utility of the method of the invention as serum obtained.

### EXAMPLE 1. Preparation of serums.

Different serums and combinations thereof were obtained as follows:
Venous blood was obtained from five (5) donors; three men and two women healthy volunteers included in this study or determination, with a mean age ± SD , 31.6 ± 10.9 years . Inclusion criteria were healthy adults who freely agreed to participate in the study and the exclusion criteria were having a medical history of not having suffered any haematological disease or blood derived disease, or not have been treated with any medication that may affect or alter the function of any element or hematic component, especially platelets and clotting factors for a minimum of 2 weeks prior to the test. Blood was collected in tubes of 9 ml (Vacuette , ref.455322) containing 3.2 % sodium citrate as an anticoagulant, and 9 ml tubes (Terumo, VENOSAFE ref. VF- 109SP) voids without any content or additive or anticoagulant. Four tubes were obtained per donor, a total of 36 ml of peripheral blood. After the removal of blood to samples (tubes) with no additives or no anticoagulant ( Terumo , VENOSAFE ref. VF- 109SP) it was added CaCl2 to a final concentration in the sample of 15µmol/ml (solution at10% (2.5 mg / ml)), reaching 135 mol of total CaCl2 . The average of initial or basal platelet and leukocyte of the five donors according CBC samples was 268.6 x10 ^ 9/L and 8.6 x10 ^ 9/L, respectively. All resulting samples of the various preparations were kept for 24h at 37 ° C. After this time samples were taken for analysis and determination of cytokines and growth factors.

Samples (tubes) with sodium citrate, and sample tubes without sodium citrate and with calcium chloride were centrifuged at 150g at once for 10 min at room temperature with an angular rotor CENCOM II (JP Selecta, model 7002240), with the aim to fractionate the blood in the tubes with sodium citrate and to form clots in the tubes without sodium citrate and having calcium chloride .

For all preparations listed below, the angular rotor CENCOM II (JP Selecta, model 7002240) was used. In this rotor 150 g are equivalent to aprox. 1200 r.p.m; 450g are equivalent to aprox. 2000 r.p.m; and 1500g are equivalent to aprox. 3700 r.p.m.

PRP preparation: After the centrifugation time it was extracted from the specimen (tube) with sodium citrate all of the supernatant (which contains mostly plasma and platelets or PRP " PRP ") until the buffy coat (buffy coat) and it was deposited in a vacuum tube with no additives (sample A). 2ml of this PRP sample was extracted for analysis of cytokines and growth factors.

Preparation of platelet-poor plasma (PPP) and platelet concentrate (cPLT): It was replaced in the centrifuge the residual sample (mostly red blood cells) of the sodium citrate tubes by the "sample A", and letting clot the sample without anticoagulant for five minutes.

Both samples, sample A and the tubes without sodium citrate and with calcium chloride, were centrifuged at 450g for 10min at room temperature. After this centrifugation appeared in "Sample A" (tube) a pellet or sedimentation of platelets and platelet-poor supernatant PPP. The supernatant PPP from the "sample A" was extracted leaving in the tube a remaining 2 ml, which is a platelet concentrate (cPLT). Platelets were stirred up and disaggregation took place. Then the sample was homogenized and allowed to stand. 2ml were obtained from each of the samples, PPP and cPLT for the analysis of cytokines and growth factors.

Preparation of cytokine-rich serum or serum rich in cytokines (SRC, also named SN1): From a blood sample without anticoagulant and with 15 µmol/ml of CaCl2 (10%), previously centrifuged at 150 g, it was extracted the supernatant released from the clot formed in a centrifugation at 450g, resting much of the formed clot yet to be degraded. The supernatant was put in an empty tube without additives (sample B). Then the resting clot was centrifuged without anticoagulant at 1500g for 10 min. After this centrifugation, supernatant was removed and mixed with "Sample B", getting serums enriched with cytokines (SRC; SN1) . 2 ml were extracted for analysis of cytokines and growth factors.

Preparation of leukocyte rich plasma (LRP): In a sample of anticoagulated peripheral blood (9 ml tubes (Vacuette , ref.455322) containing 3.2 % of sodium citrate) it was added 20% v/v of hydroxyethyl starch- HES (Hes Grifols " 6%) over the sample volume. The tube was allowed to stand upright for 30 min. After this time it showed sedimentation of the erythrocytes and a leukocyte supernatant. Supernatant was removed and centrifuged at 700g for 10min. Next, the supernatant was removed. A remnant of 2ml remained in the tube, and it was stirred to disaggregate the leukocytes and to homogenize the sample.

PRP releasate preparation and cPTL releasate: To a sample of PRP and to a sample of cPLT it was added calcium chloride to a final concentration of 24 µmol/ml (from a solution of CaCl2 at 10% w/w) . The tubes were allowed to stand to form a clot, and maintained for 24 hours at 37 ° C . After this time, samples were taken from each of the liquids for analysis of cytokines and growth factors. The term "Releasate" is described as the liquid or the supernatant resulting from activation of platelets and coagulation from a formed clot and fibrin degradation.

Preparation of SRC1 serum (a type or particular embodiment of SN2) derived from SRC (SN1) and PRP: It was prepared a mixture of SRC and PRP in a 1:1 ratio, and it was added calcium chloride (CaCI2 at 10%) to reach a concentration of about 24 µmol/ml over the volume of PRP to form a clot (fibrin matrix). It was proceeded with the same technique of double centrifugation (particular centrifugation pattern of the step (b) for the method of obtaining SN1, in which a gravity force from 400 g to 2500 g is applied) and removal of supernatant used to make the SCR. That is, 10 min centrifugation at 450g, removal of the supernatant, 10 min centrifugation of resting clot at 1500g, extraction of the supernatant and mix of both. This technique allowed breaking the clot (fibrin matrix) by applying particular gravity forces in a discontinuous mode. The sample was allowed to rest for 24 hours at 37 ° C, and a sample was taken for analysis of cytokines and growth factors.

Preparation of SRC2 (a type or particular embodiment of SN2) serum derived from SRC (SN1) and cPLT . It was prepared a mixture of cPLT and SRC in a 1:1 ratio, and Sample was mixed and CPLT SRC in a 1:1 ratio and was added calcium chloride (CaCl2) at 10% until a concentration of about 24 µmol/ml over the volume of cPLT. It was allowed to stand to form a clot (fibrin matrix). Then it was proceeded with the same technique of double centrifugation (particular centrifugation pattern of step (b) for the method of obtaining SN1, in which a gravity force from 400 g to 2500 g is applied) and removal of supernatant used to make the SCR. That is, 10 min centrifugation at 450g, removal of the supernatant, 10 min centrifugation of resting clot at 1500g, extraction of the supernatant and mix of both. This technique allowed breaking the clot (fibrin matrix) by applying particular gravity forces in a discontinuous mode. The sample was allowed to rest for 24 hours at 37 °C, and a sample was taken for analysis of cytokines and growth factors.

Preparation of SRC3 (a type or particular embodiment of SN2) serum derived from PRL+ SRC (SN1) + PPP. A mixture of SRC, PRL and PPP was prepared in a ratio of 1:1:1. It was then added calcium chloride (CaCI2 at 10%) until a concentration of 24 µmol/ml was reached over the sum of the sample volumes of PRL and PPP. The mixture was allowed to stand until the formation of a clot (fibrin matrix). Then it was proceeded with the same technique of double centrifugation (particular centrifugation pattern of step (b) for the method of obtaining SN1, in which a gravity force from 400 g to 2500 g is applied) and removal of supernatant used to make the SCR. That is, 10 min centrifugation at 450g, removal of the supernatant, 10 min centrifugation of resting clot at 1500g, extraction of the supernatant and mix of both. This technique allowed breaking the clot (fibrin matrix) by applying particular gravity forces in a discontinuous mode. The sample was allowed to rest for 24 hours at 37 °C, and a sample was taken for analysis of cytokines and growth factors.

Preparation of SRC4 (a type or particular embodiment of SN2) serum derived from SRC (SN1) + PRP + PRL: A mixture of SRC, PRP and PRL was prepared in a ratio of 1:1:1. It was then added calcium chloride (CaCI2 at 10%) until a concentration of 24 µmol/ml was reached over the sum of the sample volumes of PRP and PRL. The mixture was allowed to stand until the formation of a clot (fibrin matrix). Then it was proceeded with the same technique of double centrifugation (particular centrifugation pattern of step (b) for the method of obtaining SN1, in which a gravity force from 400 g to 2500 g is applied) and removal of supernatant used to make the SCR. That is, 10 min centrifugation at 450g, removal of the supernatant, 10 min centrifugation of resting clot at 1500g, extraction of the supernatant and mix of both. This technique allowed breaking the clot (fibrin matrix) by applying particular gravity forces in a discontinuous mode. The sample was allowed to rest for 24 hours at 37 °C, and a sample was taken for analysis of cytokines and growth factors.

Preparation of SRC5 (a type or particular embodiment of SN2) serum derived from cPLT + SRC (SN1) + PRL: A mixture of SRC, CPLT and PRL in a ratio of 1:1:1 was prepared. It was then added calcium chloride (CaCI2 at 10%) until a concentration of 24 µmol/ml was reached over the sum of sample volumes of PRL and cPLT. The mixture was allowed to stand until the formation of a clot (fibrin matrix). Then it was proceeded with the same technique of double centrifugation (particular centrifugation pattern of step (b) for the method of obtaining SN1, in which a gravity force from 400 g to 2500 g is applied) and removal of supernatant used to make the SCR. That is, 10 min centrifugation at 450g, removal of the supernatant, 10 min centrifugation of resting clot at 1500g, extraction of the supernatant and mix of both. This technique allowed breaking the clot (fibrin matrix) by applying particular gravity forces in a discontinuous mode. The sample was allowed to rest for 24 hours at 37 °C, and a sample was taken for analysis of cytokines and growth factors.

### EXAMPLE 2: Cytokine levels in different cells and serums obtainable by the method of the invention.

The amount of platelets and white blood cells in both peripheral blood, and in the samples was performed with a differential automated analyzer CELL-DYN 1700 (Abbott Diagnostics Division, Santa Clara, CA).

The panel of cytokines and growth factors described in Table 1 and 2 was determined by ELISA (Enzyme-linked immunosorbent assay) using human Quantikine kits (R & D Systems, Minneapolis, Minnesota). Several cytokines were measured in different serums obtainable by the method of the invention (Those of example 1).

In Tables 1 and 2 there are showed the values of different cytokines, growth factors and cells present in each of the analyzed serums. In these tables it is observed that cytokine-rich serums obtained by the method of the invention (SRC, SRC1 to SCR5) exhibit a higher cytokine concentration compared to plasma, PRP and PRP Releasate, and cPLT Releasate.

**Table 1: Concentrations of some cytokines in various plasma derivatives and in a SRC.**

| | **Plasma** | **PRP** | **PRP Releasate*** | **cPLT Releasate** | **SRC (SN1)** |
|---|---|---|---|---|---|
| **initial platelet** average 268,6 x10^9/L | | | | | |
| **initial WBC** average 8,6 x10^9/L | | | | | |
| **Platelets Product** x10^9/L | 24,67 | 50,33 | 15,66 | 20,8 | 21,35 |
| **leukocyte Product** x10^9/L | 0,08 | 0,07 | 0,22 | 0,45 | 0,14 |
| **TGF-β1 ng/mL** | 1,8 | 5,5 | 18,8 | 28,7 | 34,9 |
| **FGF - pg/mL** | 0,05 | 0,05 | 0,06 | 0,08 | 0,05 |
| **IGF-1 ng/mL** | 209 | 204 | 232 | 239 | 292 |
| **HGF** - **ng/mL** | 1,8 | 1,7 | 2,1 | 3,2 | 4,2 |
| **EGF - pg/ml** | 2,5 | 26,87 | 52,5 | 60,1 | 71,6 |
| **VEGF - pg/ml** | 20,3 | 25,6 | 118,3 | 135,4 | 205 |
| **IL-1 β -pg/ml** | Not detectable | Not detectable | Not detectable | Not detectable | Not detectable |
| **IL-6 -pg/ml** | 2,6 | 1,34 | 3,54 | 4,66 | 2,33 |
| **IL-8** - **pg/ml** | 2,27 | 2,7 | 2,4 | 5,5 | 1,6 |
| **TNF-α** - **pg/ml** | 1,7 | 1,33 | 4,8 | 4,7 | 4,4 |
| **IL-10** - **pg/ml** | No detectable | No detectable | 4,8 | 5,5 | 6,1 |
| **PDGF - ng/mL** | 1,2 | 3,8 | 30,2 | 38,1 | 40 |
| **BMP** - **pg/mL** | 26,6 | 28,1 | 24,3 | 32,4 | 28,8 |
| **Fibrinogen -g/l** | 2,67 | 2,63 | Not detectable | Not detectable | Not detectable |
| **Thrombin-U/ml** | Not detectable | Not detectable | 11 nM | 15 nM | 20 nM |
| | | | 5,5 U/ml | 7,5 U/ml | 10 U/ml |
| **Factor VII-U/dl** | 148,66 | 143,3 | 155,3 | 166,6 | 196,4 |
| **Von Willebrand Factor** - **U/dl** | 127,35 | 126 | 122 | 138 | 121 |

| | | | | | |
|---|---|---|---|---|---|
| WBC: white blood cells; TGF- β1: tumoral growth factor- β1; FGF: fibroblast growth factor; IGF: insulin growth factor; HGF: hepatocyte growth factor; EGF: epidermal growth factor; VEGF: vascular endothelial growth factor; IL-1 β: interleukin-1 β; IL-6: interleukin-6; IL-8: interleukin-8; TNF-α: tumoral necrosis factor- α; IL-10: interleukin-10; PDGF: platelet-derived growth factor; BMP: bone morphogenic protein. (*): Liquid similar to the liquid embedded in a platelet clot obtained (or platelet in fibrin matrix) when blood is let to clot without anticoagulants (also named platelet rich fibrin). | | | | | |

**Table 2: Concentrations of different cytokines in various serum according to the invention.**

| | **SRC+ PRP (SRC1)** | **SRC+ cPTL (SCR2)** | **SRC+PRL+ PPP (SCR3)** | **SRC+ PRP+ PRL (SCR4)** | **SRC+cPTL+ PRL (SCR5)** |
|---|---|---|---|---|---|
| **initial platelet** Media 268,6 x10^9/L | | | | | |
| **initial WBC** Media 8,6 x10^9/L | | | | | |
| **Platelets Product x10^9/L** | 42,55 | 67,2 | 33,72 | 40,84 | 70,4 |
| **Leukocyte Product x10^9/L** | 0,28 | 0,48 | 6,46 | 7,10 | 7,33 |
| **TGF-β1 ng/mL** | 100,5 | 132,2 | 102,1 | 155,4 | 176,8 |
| **FGF - pg/mL** | 0,04 | 0,06 | 0,08 | 0,06 | 0,07 |
| **IGF-1 ng/mL** | 208 | 277 | 210 | 285 | 315 |
| **HGF - ng/mL** | 10,7 | 28,4 | 15,3 | 28,5 | 42,2 |
| **EGF - pg/ml** | 89,2 | 110,4 | 81,5 | 125,7 | 177,8 |
| **VEGF - pg/ml** | 288 | 578,7 | 216,7 | 386,2 | 637,8 |
| **IL-1 β - pg/ml** | Not detectable | 8,7 | 14,2 | 16,7 | 18,3 |
| **IL-6 - pg/ml** | 3,82 | 5,4 | 35,4 | 30,1 | 38,7 |
| **IL-8 - pg/ml** | 2,8 | 3,4 | 62,2 | 68,1 | 76,4 |
| **TNF-α - pg/ml** | 3,2 | 4,5 | 4,2 | 4,7 | 5,7 |
| **IL-10 - pg/ml** | 6,3 | 7,2 | 22,6 | 24,8 | 26,5 |
| **PDGF - ng/mL** | 55,8 | 158,7 | 155,7 | 159,3 | 215,1 |
| **BMP - pg/mL** | 28,5 | 30,1 | 44,2 | 48,4 | 62,7 |
| **Fibrinogen -g/l** | Not detectable | Not detectable | Not detectable | Not detectable | not detectable |
| **Thrombin - U/ml** | 30 nM 15 U/ml | 80 nM 40 U/ml | 20 nM 10 U/ml | 33 nM 16 U/ml | 84 nM 42 U/ml |
| **Factor VII-U/dl** | 222,7 | 276,2 | 192,1 | 232,3 | 298,8 |
| **Von Willebrand Factor - U/dl** | 145 | 228 | 122 | 156 | 236 |

| | | | | | |
|---|---|---|---|---|---|
| WBC: white blood cells; TGF- β1: tumoral growth factor- β1; FGF: fibroblast growth factor; IGF: insulin growth factor; HGF: hepatocyte growth factor; EGF: epidermal growth factor; VEGF: vascular endothelial growth factor; IL-1 β: interleukin-1 β; IL-6: interleukin-6; IL-8: interleukin-8; TNF-α: tumoral necrosis factor- α; IL-10: interleukin-10; PDGF: platelet-derived growth factor; BMP: bone morphogenic protein. | | | | | |

Therefore, these tables show that the method of the invention is useful for the production of serum having high levels of cytokines from an isolated sample. These serums, due to the high levels of cytokines, have diverse and multiple uses as illustrated in the examples below.

Data of Tables 1 and 2 are illustrated in FIG. 1, FIG. 2, FIG. 3, FIG, 4 and FIG. 5. There, for each of the compositions there are depicted the concentrations of some of the cytokines as indicated in the brief description of the drawings. In each of the FIGs. 1 to 5, each bar representing a type of tested composition for a specified cytokine or compound (S) indicated in the X-axis is depicted in the following order from left to right: Plasma, PRP, PRP Releasate, cPLT releasate, SRC, SRC+PRP, SRC+cPLT, SRC+PRL, SRC+PRP+PRL, SRC+cPLT+PRL.

### EXAMPLE 3: Demonstrative and comparative test of several doses of human SRC obtainable by the method of the invention. Action on bone marrow cell proliferation.

By means of this test it was determined the degree of proliferation of bone marrow cells with different doses of cytokine-rich serum (SCR) obtained by the method described in this patent. This example illustrates the effects of serum SCR2 (a type or particular embodiment of SN2), since it seems the most appropriate due to the cytokine concentration and to the balance between cytokines (types of cytokines).

Bone marrow cells were grown under three different culture conditions, and uniformly seeded in Petri dishes with a density of 200 cells /cm2 for 15 days. Samples of bone marrow cells were obtained from bone marrow aspirates from 3 patients/donors. The aspirates were for the regeneration of bone tissue, from men aged 28-44 years with a mean age of 36 years. Consent was obtained from these patients/ donors in accordance with the ethical standards of the local ethics committee. After collecting the adherent cells, they were counted and cell expression studies of the cell surface antigen was performed.

Cell viability was determined by trypan blue exclusion (Sigma-Aldrich), and the cells were counted using a Neubauer chamber (Marienfeld GmbH, Lauda-Königshofen, Germany).

Cytokine-rich serum (SCR2) demonstrated to be a significantly better medium than fetal calf serum (FCS) for the proliferation of bone marrow cells in all of the SRC2 studied concentrations (FIG.s 6A, 6B and 7). These data are directly derivable from FIG. 6A, wherein the number of bone marrow cells (Y-axis) at various times (T in days, d), are depicted. The cells were grown in basal media supplemented with fetal calf serum (FCS or Foetal calf serum; the control) or supplemented with different concentrations ((10, 20, 50 and 80% w/w) of a cytokine-rich serum, SRC2, of the invention. Pannel (A) shows the number of cells along time in the different tested conditions (different % of the serum of the invention). In Pannel (B) it is shown the cumulative duplication of cells contacted with the different mediums.

In order to make more visible the effect of the serum of the invention, in FIG. 7, there are depicted different optical microscopic images (100X) of the bone marrow cells cultivated as indicated in FIG. 6. Cell morphology is maintained in all assays. Increasing amounts of SCR2 increase the total number of cells per field (cell density).

Immunophenotyping of cells was performed by flow cytometry (FACScan flow cytometer - CellQuest software (Becton Dickinson)), using the following conjugated mouse antihuman antibodies: CD45, CD34, CD90, CD166, CD105 (Immunotech-Coulter). Results are depicted in FIG.s 8 and 9.

FIG. 8 is a diagram with the phenotype analysis of the bone marrow cells submitted to the conditions as indicated in FIGs. 6 and 7. The phenotype was determined by the expression levels of several membrane antigens or markers for each of the assayed conditions (X-axis). The Y-axis shows the number of cells of each phenotype. CD45 leukocyte marker; hematopoietic marker CD34; mesenchyme lineage marker CD90; CD166 stromal lineage marker; and endothelial lineage marker CD105.

FIG. 9 represents the kinetics evolution of the different cell populations in cultures of bone marrow cells along the time (X-axis, T in days (d)). In the Y-axis it is indicated the percentage of each cell type with respect of the total cells in the medium. It can be seen that along time the percentage of leukocytes (represented by the cells with CD45 marker) in the initial sample (from the bone marrow aspirates) is progressively reduced. On the other side, the rest of the cell types increase. These cells that became majority correspond to progenitor cells or stem cells also present in the initial bone marrow sample. Accordingly, with the serum of the invention these progenitor cells could proliferate. Consequently, cell proliferation capacity of the bone marrow by means of these cell types was maintained making possible the regeneration of bone tissue.

As base medium it was used the Dulbecco's modified medium from Eagle that has low glucose levels (DMEM-LG, Cambrex, Walkersville) and having 4 mM L-Glutamine (PAA, Pasching, Austria) and 0.05 units of penicillin and 0, 05 g of streptomycin (PAA). The base medium was supplemented with:
(a) base medium with 10% fetal calf serum (FCS - Cambrex)
(b) base medium with 10% SCR2
(c) base medium with 20% SCR2
(d) base medium with 50% SCR2
(e) basic medium with 80% SCR2

As indicated in FIG. 7, the morphology and expansion of bone marrow cells (x100 magnification) in different percentages of SRC2 was higher than that of the cells with only FCS. The cells in SCR2 medium maintained their morphology during the 15 days of culture. In addition, duplication of population and the cell expansion rate significantly increased up to the saturation medium containing SRC2 in all samples after 12 days of culture. These results revealed that the cytokine-rich serums (SRC2) derived from blood contained abundant growth and differentiation factors, being useful as potential proliferation elements of bone marrow cells.

Expression of cell surface markers analyzed population was positive in all culture conditions (FIG.s 8 and 9).

### EXAMPLE 4: Use of the serums of the invention for tissue regeneration.

Three analysis were performed using the serum of the invention for regenerating several types of damaged tissue of.

In a patient (52 years old) with craniofacial trauma for 12 years, massive bone loss, chronic pain, oral dysfunction and osteomyelitis, a bilaterally sinus lift was performed with multiple infiltrations (injections) containing tricalcium phosphate and SRC2 around the lesions. In FIG. 10 it is shown as bone regeneration occurred in a short period of time (6 months) due to infiltration with SRC2. Panel A is the MRI image taken after the sinus lift, and pannel B shows the zone (area) treated with infiltrations for 6 months (one infiltration per month).

In another male patient (32 years old) suffering from scaphoid fracture and ulna of the right hand, which was subjected to immobilization of a member for a while without success, it was carried out surgery fixation using osteosynthesis material, and a resection of the distal ulna was associated. Postsurgical infiltrations were performed in the ulna with cytokine-rich serum (SRC2) supporting the regeneration of bone tissue (four doses of 6ml in the affected area every twenty days). As shown in FIG. 11, the bone regeneration region was satisfactory, and the wrist mobility and muscular strength was restored in a satisfactory manner. The patient became asymptomatic and could make normal life.

The third patient is a woman (24 years old), which presents a keloid for two years. Keloid scars are an anomaly of healing and are defined as an abnormal scar that grows beyond the limit of the original surface of the skin injury. Although anyone can form a keloid scar some ethnic groups are at higher risk of developing them. It is not fully understand the etiology or how keloid scars are formed. Skin trauma seems to be the most common cause, although sometimes they can be formed and there seems no apparent causal relationship. There is a genetic predisposition or inherited genetic factor to develop keloid scars. Other causes include the deficiency or excess production and release of melanin (melanogenesis) by melanocytes in the skin, decreased rates of mature collagen and increased amounts of soluble collagen, or blockage of blood vessels with a consequent lack of oxygen in the dermal and epidermal tissue. It was established a treatment with three doses of infiltration into the lesion with 4 ml of cytokine-rich serum (SRC2) every 10 days. As shown in FIG. 12 tissue regeneration and proper healing clearly takes place. No adverse reactions were observed and no skin irritation of the treated area.

### REFERENCES CITED IN THE APPLICATION

- US 20050252867
- Isogai et al. in "Cytokine-Rich Autologous Serum System for Cartilaginous Tissue Engineering", Annals of Plastic Surgery - 2008, Vol. NO. 60, pp.: 703-709.
- Su et al. in "In vitro release of growth factors from platelet-rich fibrin (PRF): a proposal to optimize the clinical applications of PRF", Oral Surg Oral Med Oral Pathol Oral radiol Endod - 2009, Vol. No. 108, pp.: 56-61.

## Claims

1. A method for preparing a serum comprising cytokines and coagulation factors, the method comprising the following steps:
(a) Submitting an isolated biological sample comprising platelets and/or leukocytes to a gravity force comprised from 100 g to 200 g to obtain a first clot and a supernatant;
(b) Submitting only the clot or, alternatively, the clot and supernatant of step (a) to a gravity force comprised from 400 g to 2500 g to obtain a supernatant SN1; wherein the gravity force is discontinuously applied and carried out by means of:
(i) centrifuging the clot of step (a), or alternatively the clot and supernatant, at a gravity force comprised from 400 g to 500 g;
(ii) recovering the supernatant of step (i), which constitutes partially the supernatant SN1ii;
(iii) centrifuging the remaining clot at a gravity force comprised from 1500 g to 2500 g and recovering the supernatant of step (iii), which constitutes partially the supernatant SN1iii of step (b), constituting the supernatants of steps (ii) and (iii) the entire supernatant SN1; and
(c) Recovering said supernatant SN1 of step (b), which is a serum comprising cytokines and coagulation factors,
wherein the gravity force is applied by a process selected from the group consisting of centrifugation and physical-suction.

2. The method according to claim 1, wherein prior to step (b) the clot or, alternatively, the clot and supernatant of step (a) is submitted to a gravity force comprised from 1 g to 10 g.

3. The method according to any of claims 1-2, wherein after step (c) it is performed an additional step (d) of adjusting the ionic calcium to a final concentration from 1.0 µmol/ml to 50.0 µmol/ml and pH of the final solution to a pH comprised from 7.0 to 7.6, by adding an ionic calcium source to the recovered supernatant and, optionally, a pH adjusting agent.

4. The method according to any of claims 1-3, wherein gravity force is applied by centrifugation.

5. The method according to any of claims 1-4, further comprising:
- mixing the supernatant SN1 of step (c) with a composition comprising fibrinogen, platelets and/or leukocytes selected from the group consisting of a platelet-rich plasma, a platelet concentrate, a leukocyte-rich plasma, a leukocyte concentrate, a platelet-poor plasma, a plasma concentrate and mixtures or combinations thereof;
- adding to the mixture of previous step an ionic calcium source to adjust the ionic calcium to a final concentration comprised from 1.0 µmol/ml to 50.0 µmol/ml to obtain a second clot, which is a fibrin gel composition comprising platelets and/or leukocytes embedded in a fibrin matrix, said gel comprising also a serum with cytokines and coagulation factors in liquid form disposed in-between the fibrin matrix.

6. The method according to claim 5, further comprising the steps of
- submitting the second clot, which is a fibrin gel composition comprising platelets and/or leukocytes embedded in a fibrin matrix, to physical and/or chemical and/or biochemical means to break the fibrin matrix by these means to obtain a supernatant SN2; and
- recovering the supernatant SN2, which is a serum comprising cytokines and coagulation factors.

7. The method according to claim 6, wherein a gravity force comprised from 400 g to 2500 g is used as a physical means to break the fibrin matrix.

8. The method according to any of claims 6-7, wherein the physical means are centrifugation.

9. The method according to any of claims 6-8, wherein the physical means to break the fibrin matrix is a gravity force applied discontinuously by means of:
(i) centrifuging the clot formed by the platelets and/or leukocytes embedded in a fibrin matrix at a gravity force comprised from 400 g to 500 g;
(ii) recovering the supernatant of step (i), which constitutes partially the supernatant SN2; and
(iii) centrifuging the remaining clot at a gravity force comprised from 1500 g to 2500 g and recovering the supernatant of step (iii), which constitutes partially the supernatant SN2, constituting the supernatants of steps (i) and (iii) the entire supernatant SN2, which is a serum comprising cytokines and coagulation factors.

10. A serum comprising cytokines and coagulation factors obtainable by the method according to any of claims 1-4.

11. A fibrin gel composition comprising platelets and/or leukocytes embedded in a fibrin matrix, said gel comprising also a serum with cytokines and coagulation factors, obtainable by the method according to claim 5.

12. A serum comprising cytokines and coagulation factors obtainable by the method according to any of claims 6-9.

13. A pharmaceutical composition comprising the serum as defined in any of the claims 10 or 12, or comprising the fibrin gel composition as defined in claim 11 together with pharmaceutically acceptable carriers and/or excipients.

14. A serum as defined in claim 10, or alternatively, a serum as defined in claim 12, or alternatively a fibrin gel composition as defined in claim 11, or alternatively a pharmaceutical composition as defined in claim 13, for use as a medicament.

15. The serum as defined in claim 10, or alternatively, the serum as defined in claim 12, or alternatively, the fibrin gel composition as defined in claim 11, or alternatively, the pharmaceutical composition as defined in claim 13, for use in the treatment of a disease or pathology selected from the group consisting of an inflammatory pathology, a degenerative disease, a disease caused by ischemia, a vascular disease, an immunological pathological process, and trauma.

16. The serum as defined in claim 10, or alternatively, the serum as defined in claim 12, or alternatively the fibrin gel composition as defined in claim 11, or alternatively, the pharmaceutical composition as defined in claim 13, for use as an organ and/or tissue regenerative and/or reparative agent.

## Patentansprüche

1. Ein Verfahren zur Herstellung eines Serums umfassend Zytokine und Gerinnungsfaktoren, wobei das Verfahren die folgenden Schritte umfasst:
(a) Aussetzen von einer isolierten biologischen Probe umfassend Thrombozyten und/oder Leukozyten einer Schwerkraft reichend von 100 g bis 200 g, um ein erstes Gerinnsel und einen Überstand zu erhalten;
(b) Aussetzen des Gerinnsels nur oder, ersatzweise, des Gerinnsels und des Überstands des Schritts (a) einer Schwerkraft reichend von 400 g bis 2.500 g, um einen Überstand SN1 zu erhalten; wobei die Schwerkraft diskontinuierlich ausgeübt wird und wie folgt angewendet wird:
(i) schleudern des Gerinnsels des Schritts (a) oder, ersatzweise, des Gerinnsels und des Überstands bei einer Schwerkraft reichend von 400 g bis 500 g;
(ii) zurückgewinnen des Überstands des Schritts (i), welcher teilweise den Überstand SN1ii ausmacht;
(iii) schleudern des Restgerinnsels bei einer Schwerkraft reichend von 1.500 g bis 2.500 g und zurückgewinnen des Überstands des Schritts (iii), welcher teilweise den Überstand SN1iii des Schritts (b) ausmacht, wobei die Überstände der Schritte (ii) und (iii) den gesamten Überstand SN1 ausmachen; und
(c) zurückgewinnen des Überstands SN1 des Schritts (b), welcher ein Serum umfassend Zytokine und Gerinnungsfaktoren ist,
wobei die Schwerkraft durch ein Verfahren ausgewählt aus der Gruppe bestehend aus Zentrifugation und physikalischer Sogwirkung angewendet wird.

2. Das Verfahren nach Anspruch 1, wobei das Gerinnsel oder, ersatzweise, das Gerinnsel und der Überstand des Schritts (a), einer Schwerkraft reichend von 1 g bis 10 g vor dem Schritt (b) ausgesetzt wird.

3. Das Verfahren nach einem der Ansprüche 1-2, wobei ein zusätzlicher Schritt (d) bestehend in der Einstellung des Gehalts an ionischem Calcium auf eine Endkonzentration von 1,0 µmol/ml bis 50,0 µmol/ml und des pH-Werts der Endlösung auf einen pH-Wert von 7,0 bis 7,6 nach dem Schritt (c) durchgeführt wird, in dem eine Quelle ionischen Calciums und, wahlweise, ein Mittel zur Einstellung des pH-Werts dem zurückgewonnenen Überstand zugegeben wird.

4. Das Verfahren nach einem der Ansprüche 1-3, wobei die Schwerkraft durch Zentrifugation ausgeübt wird.

5. Das Verfahren nach einem der Ansprüche 1-4, weiterhin umfassend:
- mischen des Überstands SN1 des Schritts (c) mit einer Zusammensetzung umfassend Fibrinogen, Thrombozyten und/oder Leukozyten ausgewählt aus der Gruppe bestehend aus einem thrombozytenreichen Plasma, einem Thrombozytenkonzentrat, einem leukozytenreichen Plasma, einem Leukozytenkonzentrat, einem thrombozytenarmen Plasma, einem Plasmakonzentrat und Mischungen oder Kombinationen davon;
- beimischen von einer Quelle ionischen Calciums der Mischung des vorherigen Schritts, um das ionische Calcium bis zu einer Endkonzentration reichend von 1,0 µmol/ml bis 50,0 µmol/ml einzustellen, um ein zweites Gerinnsel zu erhalten, welches eine Fibringelzusammensetzung umfassend Thrombozyten und/oder Leukozyten ist, die in einer Fibrinmatrix eingebettet sind, wobei das Gel auch ein Serum mit sich dazwischen in der Fibrinmatrix befindenden Zytokinen und Gerinnungsfaktoren in flüssiger Form umfasst.

6. Das Verfahren nach Anspruch 5, weiterhin umfassend folgende Schritte:
- aussetzen des zweiten Gerinnsels, welches eine Fibringelzusammensetzung ist, die Thrombozyten und/oder Leukozyten eingebettet in einer Fibrinmatrix umfasst, physikalischen und/oder chemischen und/oder biochemischen Mitteln, um die Fibrinmatrix auf diese Weise zu brechen, um einen Überstand SN2 zu erhalten; und
(c) zurückgewinnen des Überstands SN2, welcher ein Serum umfassend Zytokine und Gerinnungsfaktoren ist.

7. Das Verfahren nach Anspruch 6, wobei eine Schwerkraft reichend von 400 g bis 2.500 g als physikalisches Mittel angewendet wird, um die Fibrinmatrix zu brechen.

8. Das Verfahren nach einem der Ansprüche 6-7, wobei das physikalische Mittel Zentrifugation ist.

9. Das Verfahren nach einem der Ansprüche 6-8, wobei das physikalische Mittel zum Brechen der Fibrinmatrix eine Schwerkraft ist, die diskontinuierlich wie folgt ausgeübt wird:
(i) schleudern des aus den in einer Fibrinmatrix eingebetteten Thrombozyten und/oder Leukozyten bestehenden Gerinnsels einer Schwerkraft reichend von 400 g bis 500 g;
(ii) zurückgewinnen des Überstands des Schritts (i), welcher teilweise den Überstand SN2 ausmacht; und
(iii) schleudern des Restgerinnsels einer Schwerkraft reichend von 1.500 g bis 2.500 g und zurückgewinnen des Überstands des Schritts (iii), welcher teilweise den Überstand SN2 ausmacht, wobei die Überstände der Schritte (i) und (iii) den gesamten Überstand SN2 ausmachen, welcher ein Serum umfassend Zytokine und Gerinnungsfaktoren ist.

10. Ein Serum umfassend Zytokine und Gerinnungsfaktoren, welches mittels des Verfahrens nach einem der Ansprüche 1-4 erhalten werden kann.

11. Eine Fibringelzusammensetzung umfassend Thrombozyten und/oder Leukozyten, die in einer Fibrinmatrix eingebettet sind, wobei das Gel auch ein Serum mit Zytokinen und Gerinnungsfaktoren umfasst, welches mittels des Verfahrens nach Anspruch 5 erhalten werden kann.

12. Ein Serum umfassend Zytokine und Gerinnungsfaktoren, welches mittels des Verfahrens nach einem der Ansprüche 6-9 erhalten werden kann.

13. Eine pharmazeutische Zusammensetzung umfassend das Serum wie in einem der Ansprüche 10 oder 12 definiert, oder umfassend die Fibringelzusammensetzung wie in Anspruch 11 definiert zusammen mit pharmazeutisch akzeptablen Trägersubstanzen und/oder Hilfsstoffen.

14. Ein Serum wie in Anspruch 10 definiert oder, ersatzweise, ein Serum wie in Anspruch 12 definiert oder, ersatzweise, eine Fibringelzusammensetzung wie in Anspruch 11 definiert oder, ersatzweise, eine pharmazeutische Zusammensetzung wie in Anspruch 13 definiert, zur Verwendung als Arzneimittel.

15. Das Serum wie in Anspruch 10 definiert oder, ersatzweise, das Serum wie in Anspruch 12 definiert oder, ersatzweise, die Fibringelzusammensetzung wie in Anspruch 11 definiert oder, ersatzweise, die pharmazeutische Zusammensetzung wie in Anspruch 13 definiert, zur Verwendung in der Behandlung von einer Krankheit oder einer Pathologie ausgewählt aus der Gruppe bestehend aus einer entzündlichen Pathologie, einer degenerativen Krankheit, einer durch Ischämie verursachten Krankheit, einer Gefäßkrankheit, einem immunologischen pathologischen Prozess, und einem Trauma.

16. Das Serum wie in Anspruch 10 definiert oder, ersatzweise, das Serum wie in Anspruch 12 definiert oder, ersatzweise, die Fibringelzusammensetzung wie in Anspruch 11 definiert oder, ersatzweise, eine pharmazeutische Zusammensetzung wie in Anspruch 13 definiert, zur Verwendung als organ- bzw. geweberegenerierendes und/oder als organ- bzw. gewebereparierendes Mittel.

## Revendications

1. Un procédé de préparation d'un sérum comprenant des cytokines et des facteurs de coagulation, le procédé comprenant les étapes suivantes :
(a) Soumettre un échantillon biologique isolé comprenant des plaquettes sanguines et/ou des leucocytes à une force de gravité comprise de 100 g à 200 g afin d'obtenir un premier caillot et un surnageant ;
(b) Soumettre seulement le caillot ou, alternativement, le caillot et le surnageant de l'étape (a) à une force de gravité comprise de 400 g à 2.500 g pour obtenir un surnageant SN1 ; dans lequel la force de gravité est appliquée de façon discontinue et effectuée par :
(i) centrifugation du caillot de l'étape (a) ou, alternativement, du caillot et du surnageant, sous une force de gravité comprise de 400 g à 500 g ;
(ii) récupération du surnageant de l'étape (i), qui constitue en partie le surnageant SN1ii;
(iii) centrifugation du caillot restant sous une force de gravité comprise de 1.500 g à 2.500 g et récupération du surnageant de l'étape (iii), qui constitue en partie le surnageant SN1iii de l'étape (b), constituant les surnageants des étapes (ii) et (iii) la totalité du surnageant SN1 ; et
(c) Récupérer ledit surnageant SN1 de l'étape (b), qui est un sérum comprenant des cytokines et des facteurs de coagulation,
dans lequel la force de gravité est appliquée par un processus choisi dans le groupe constitué de la centrifugation et l'aspiration physique.

2. Le procédé selon la revendication 1, dans lequel avant de l'étape (b) le caillot ou, alternativement, le caillot et le surnageant de l'étape (a), est soumis à une force de gravité comprise de 1 g à 10 g.

3. Le procédé selon l'une quelconque des revendications 1-2, dans lequel après l'étape (c) une étape additionnelle (d) est effectuée consistant à ajuster le calcium ionique jusqu'à une concentration finale de 1,0 µmol/ml à 50,0 µmol/ml et le pH de la solution finale à un pH compris de 7,0 à 7,6, en ajoutant une source de calcium ionique au surnageant récupéré et, facultativement, un agent d'ajustement du pH.

4. Le procédé selon l'une quelconque des revendications 1-3, dans lequel la force de gravité est appliquée par centrifugation.

5. Le procédé selon l'une quelconque des revendications 1-4, comprenant en outre :
- mélanger le surnageant SN1 de l'étape (c) avec une composition comprenant du fibrinogène, des plaquettes sanguines et/ou des leucocytes choisie dans le groupe constitué d'un plasma riche en plaquettes, un concentré de plaquettes, un plasma riche en leucocytes, un concentré de leucocytes, un plasma pauvre en plaquettes, un concentré de plasma et des mélanges ou des combinaisons de ceux-ci ;
- ajouter au mélange de l'étape précédente une source de calcium ionique afin d'ajuster le calcium ionique jusqu'à une concentration finale comprise de 1,0 µmol/ml à 50,0 µmol/ml afin d'obtenir une second caillot, qui est une composition de gel de fibrine comprenant des plaquettes sanguines et/ou des leucocytes intégrés dans une matrice de fibrine, ledit gel comprenant aussi un sérum avec des cytokines et des facteurs de coagulation sous forme liquide intercalé dans la matrice de fibrine.

6. Le procédé selon la revendication 5, comprenant en outre les étapes de :
- soumettre le second caillot, qui est une composition de gel de fibrine comprenant des plaquettes sanguines et/ou des leucocytes intégrés dans une matrice de fibrine, à un moyen physique et/ou chimique et/ou biochimique pour rompre la matrice de fibrine en utilisant ces moyens-ci afin d'obtenir un surnageant SN2 ; et
(c) récupérer le surnageant SN2, qui est un sérum comprenant des cytokines et des facteurs de coagulation.

7. Le procédé selon la revendication 6, dans lequel une force de gravité comprise de 400 g à 2.500 g est utilisée comme moyen physique pour rompre la matrice de fibrine.

8. Le procédé selon l'une quelconque des revendications 6-7, dans lequel le moyen physique est la centrifugation.

9. Le procédé selon l'une quelconque des revendications 6-8, dans lequel le moyen physique pour rompre la matrice de fibrine est une force de gravité appliquée de façon discontinue moyennant :
(i) centrifugation du caillot formé par les plaquettes sanguines et/ou leucocytes intégrés dans une matrice de fibrine sous une force de gravité comprise de 400 g à 500 g ;
(ii) récupération du surnageant de l'étape (i), qui constitue en partie le surnageant SN2 ; et
(iii) centrifugation du caillot restant sous une force de gravité comprise de 1.500 g à 2.500 g et récupération du surnageant de l'étape (iii), qui constitue en partie le surnageant SN2, constituant les surnageants des étapes (i) et (iii) la totalité du surnageant SN2, qui est un sérum comprenant des cytokines et des facteurs de coagulation.

10. Un sérum comprenant des cytokines et des facteurs de coagulation qui peut être obtenu par le procédé selon l'une quelconque des revendications 1-4.

11. Une composition de gel de fibrine comprenant des plaquettes sanguines et/ou des leucocytes intégrés dans une matrice de fibrine, ledit gel comprenant aussi un sérum avec des cytokines et des facteurs de coagulation qui peut être obtenu par le procédé selon la revendication 5.

12. Un sérum comprenant des cytokines et des facteurs de coagulation qui peut être obtenu par le procédé selon l'une quelconque des revendications 6-9.

13. Une composition pharmaceutique comprenant le sérum tel que défini dans l'une quelconque des revendications 10 ou 12, ou comprenant la composition de gel de fibrine telle que définie dans la revendication 11 conjointement avec des véhicules et/ou des excipients pharmaceutiquement acceptables.

14. Un sérum tel que défini dans la revendication 10 ou, alternativement, un sérum tel que défini dans la revendication 12 ou, alternativement, une composition de gel de fibrine telle que définie dans la revendication 11 ou, alternativement, une composition pharmaceutique telle que définie dans la revendication 13, pour l'utilisation comme médicament.

15. Le sérum tel que défini dans la revendication 10 ou, alternativement, le sérum tel que défini dans la revendication 12 ou, alternativement, la composition de gel de fibrine telle que définie dans la revendication 11 ou, alternativement, la composition pharmaceutique telle que définie dans la revendication 13, pour l'utilisation dans le traitement d'une maladie ou pathologie choisie dans le groupe constitué d'une pathologie inflammatoire, une maladie dégénérative, une maladie causée par l'ischémie, une maladie vasculaire, un processus pathologique immunitaire, et un traumatisme.

16. Le sérum tel que défini dans la revendication 10 ou, alternativement, le sérum tel que défini dans la revendication 12 ou, alternativement, la composition de gel de fibrine telle que définie dans la revendication 11 ou, alternativement, une composition pharmaceutique telle que définie dans la revendication 13, pour l'utilisation comme agent de régénération et/ou de réparation d'organes et/ou de tissus.
